(19) **Europäisches Patentamt / European Patent Office / Office européen des brevets**

(11) **EP 4 353 838 A1**

(12) **EUROPEAN PATENT APPLICATION**

(43) Date of publication:
**17.04.2024 Bulletin 2024/16**

(21) Application number: **22306522.8**

(22) Date of filing: **10.10.2022**

(51) International Patent Classification (IPC):
**C12Q 1/6886** (2018.01)

(52) Cooperative Patent Classification (CPC):
**C12Q 1/6886; G16H 10/40; G16H 50/30;**
C12Q 2600/118; C12Q 2600/156

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Assistance Publique - Hôpitaux de Paris**
  **75610 Paris Cedex 12 (FR)**
• **Université Paris-Est Créteil Val de Marne**
  **94000 Créteil (FR)**
• **Universite Sorbonne Paris Nord**
  **93430 Villetaneuse (FR)**

(72) Inventors:
• **NAHON, Pierre**
  **93000 BOBIGNY (FR)**
• **AUDUREAU, Etienne**
  **94000 CRÉTEIL (FR)**

(74) Representative: **Flesselles, Bruno F.G.**
**BF IP**
**36 rue Jean de la Fontaine**
**75016 Paris (FR)**

Remarks:
The complete document including Reference Table(s) and the Sequence Listing(s) can be downloaded from the EPO website

(54) **METHOD FOR HEPATOCELLULAR CARCINOMA RISK STRATIFICATION**

(57) The invention relates to a method for hepatocellular carcinoma (HCC) risk stratification in patients, in particular with advanced chronic liver disease or cirrhosis using combinations of single nucleotide polymorphisms (SNPs), alone or with blood markers.

EP 4 353 838 A1

**Description**

The invention relates to a method for hepatocellular carcinoma (HCC) risk stratification in patients, in particular with advanced chronic liver disease (acid) or cirrhosis using combinations of single nucleotide polymorphisms (SNPs), alone or with blood markers.

Patients with cirrhosis are eligible for HCC surveillance by means of semiannual liver ultrasound (US) to detect early tumors accessible for curative procedure. However, such surveillance method has a low sensitivity below 50%. In a population where about 1.5-2% of patients will develop an HCC annually, it is important to identify the patients with a particularly high incidence, *i.e.* to predict whether a patient belongs to the sub- population with an HCC occurrence higher than 3% each year. In other words, it is important to detect patients that have a higher risk of developing HCC in the coming years (as compared to the general risk in the population of patients for which a follow-up is performed). Therefore, it is desired to stratify the population of patients, in at least two groups, one group of patients having a below than average risk of developing HCC within a given period of time (generally one year), and one risk of patients having a higher than average risk of developing HCC within such period of time.

Appropriate treatment and/or more focused or specific detection methods (including deeper investigation such as liver MLRI or measure of other circulating biomarkers) can be specifically proposed to these patients in a cost-effective manner, while maintaining the regular protocol (liver ultrasound every 6 months) for the patients not identified as belonging to this population at increased risk.

The risk of hepatocellular carcinoma (HCC) development in patients with advanced chronic liver diseases (ACLD) may be influenced by genetic factors [1]. Several single nucleotide polymorphisms (SNPs) have been reported as susceptibility loci of HCC, in particular rs738409 (PNPLA3) [2], rs58542926 (TM6SF2) [3], rs187429064 (TM6SF2) [38], rs72613567 (HSD17B13) [4], rs429358 (APOE) [38] and rs641738 (MBOAT7) [5]. All of them were initially identified through genome-wide association studies (GWAS) exploring non-alcoholic fatty liver disease (NAFLD) [6, 39] and/or alcoholic liver disease (ALD) [7]. They were subsequently tested, alone or combined into genetic risk scores (GRS), in case-control studies encompassing ACLD patients without active viral replication that was complicated or not by HCC [8-10] (ref Innes). Although the biological consequences of these SNPs are not fully understood, they seem to affect lipid metabolism without a demonstrated direct effect on the liver carcinogenic process [11]. More recently, the first GWAS dedicated to HCC in individuals of European ancestry was performed [12], and identified an additional variant modulating the Wnt-β-catenin pathway, WNT3A-WNT9A, specifically associated with liver cancer in individuals with ALD. Nevertheless, beyond these associations, the ability of this genetic information to predict the development of HCC and refine liver cancer risk stratification in patients with ACLD is currently unknown.

Semi-annual HCC surveillance using liver ultrasound (US) examination in patients with cirrhosis is endorsed by all international societies [13]. However, this monitoring is affected by the low sensitivity of US to detect small HCC [14]. Improving the efficacy of HCC surveillance implies the use of more sophisticated tools, but this strategy faces cost-effectiveness issues [13]. HCC risk stratification will play a pivotal role in justifying the implementation of these costly procedures [15]; for instance, it has been shown that early HCC detection using MRI was cost-effective in patients with a yearly cancer incidence above 3% [16, 17]. This risk stratification can be easily performed using simple features encompassing routine parameters; this is particularly the case in the era of widespread use of antivirals [18], as "universal" scoring systems have been developed in patients with ACLD regardless of the cause [17, 19]. Routine clinical scores are already used for research purposes to stratify at-risk populations in the setting of clinical trials testing new performant buy costly early HCC detection procedures. In this setting, the addition of genetic information to these already performant models needs to be assessed before considering their utility for clinical practice. The aim of the present application is to demonstrate the added prognostic value to HCC prediction of using SNPs, as well as their ability to refine HCC stratification based on clinical models.

The invention is thus based on the fact that it is possible to stratify patients by using a score that is determined according to the combination of alleles of genes associated with HCC, of the patients. In a preferred embodiment, the alleles of at least two genes associated with HCC (in particular affecting lipid metabolism) are studied and an individual notation is given according to the presence of no, one or two alleles specifically associated with HCC. In a preferred embodiment, the alleles of at least three such genes, more preferably at least four such genes, more preferably at least five such genes, more preferably at least six such genes are studied and individually scored.

The genes associated with HCC present various alleles, some of which having been identified with a higher occurrence in patients who developed HCC (and herein designated as specifically associated with HCC), and some of which having being identified with a higher occurrence with patients who didn't develop HCC.

The score for each gene is preferably 0 when the patient has no allele specifically associated with HCC, 1 when the patient has one allele specifically associated with HCC and one allele that is not specifically associated with HCC, and 2 when the patient is homozygous for an allele specifically associated with HCC or contains two alleles specifically associated with HCC.

For each gene studied, a score from 0 to 2 is then obtained.

The scores of all alleles are then preferably summed up so as to obtain a final score that ranges from 0 (the patient has no alleles specifically associated with HCC for the studied genes) to 2xn (n being the number of genes studied for the patient), indicating that the patient has alleles specifically associated with HCC on both chromosomes for each studied genes.

The final score can thus be assigned to the patient for whom the increased risk of HCC is sought, and comparison of the final score with a threshold indicates whether the patient has an increased risk or not.

For instance, it is possible to consider that, if the final score is higher than 2n/3, the patient has an increased risk of having HCC. If the final score is below n/3, the patient has a lower risk (risk below the average risk of the population). If the final score is between n/3 and 2n/3, the patient has an average risk.

Patients with an increased risk can thus be submitted to a treatment or increased follow-up, including further investigations as described above.

In a first embodiment, the invention thus relates to a method for determining whether a patient has an increased risk of developing HCC (hepatocarcinoma cancer) within a given period of time, comprising

a) Determining the presence or absence of alleles specifically associated with HCC in the genome of the patient in genes associated with HCC, wherein the alleles of at least two genes are determined,
b) Allocating a numerical value depending on the presence of none, one or two alleles specifically associated with HCC in the genome of the patient
c) Combining, in a mathematical function, all the values obtained in b) in order to obtain an end value.

The given period of time is any period of time that is of relevance for the physician. In particular, it may be one year, as this period of time is consistent with the current practice (yearly US monitoring), and it is interesting to identify those patients who would have an increased risk of having HCC between two exams. However, it is also possible to use longer period of times such as two, three or even five years, in particular to see, year after year, how the risk evolves (the risk would change according to the variation of the biological markers and/or the year, when they are used (see below). The function of c) may also include the sex (male / female) and the age of the patient, or the diabetes status of the patient.

In a preferred embodiment, the patient has cirrhosis, in particular non-viral cirrhosis. Should the patient have has viral cirrhosis in the past, such cirrhosis is either cured or under control (no viral nucleic acid can be detected in samples of the patient). Generally speaking, the methods can be performed for patients with advanced chronic liver disease.

As indicated above, such risk is increased as compared to the general risk of a population of comparable patients, i.e. patients having the same clinical condition (cirrhosis, in particular non-viral, as described above).

It is preferred when the function is an algebraic sum. However, other type of functions could be used (such as multiplication: in this case, however, the numerical value allocated in the case of absence of any allele specifically associated with HCC would be 1), but algebraic sum is preferred as it provides a discrete series of numbers for the different possible combinations (consecutive numbers if 0, 1 and 2 are selected), thus not providing too much weight to some combination of presence/absence of alleles specifically associated with HCC.

The method is performed ex *vivo,* in the sense or it doesn't include any steps of interaction with the patient's body.

The method may also include a step of comparing the end value obtained in c) to a predetermined value. It is thus possible to conclude that the patient has an increased risk of developing HCC if the end value is higher than the predetermined value. As indicated above, the predetermined value may be, in particular if the algebraic sum is used, 2n/3 with n being the number of genes for which the allele characterization has been performed. In another embodiment, the end value is such that the conclusion is that the patient doesn't have an increased risk of developing HCC. This is for example when the the end value is below than the predetermined value, and when the predetermined value is n/3.

The method herein disclosed has been developed by the inventors by following up a population of patients during a given period of time, and determining that analyzing the genetic information of the patients (determination of alleles of multiple genes associated with HCC) can lead, after proper processing of the information, to obtain a good prediction of the increased risk of occurrence of HCC within the given period of time. The predetermined value indicated above, that provides the cut-off for increased risk, may also be the value for which 25% of patients have a higher end value.

The methods herein disclosed use combination of allelic information of genes associated with HCC, alone or with other markers (biological or biochemical, clinical and/or physical markers) to provide the increased risk of developing HCC.

Among the genes that are considered to be associated with HCC, one can cite sequences and the genes of Table 1:

Table 1. Alleles associated with increased risk of developing HCC

| SNP | rs number | Allele associated with HCC risk |
|---|---|---|
| TNF G-308A | rs1800629 | A |
| TNFα G-238A | rs361525 | A |
| TNFα C-863A | rs1800630 | A |
| IL1β C-511T | rs16944 | T |
| IL1β C-31T | rs143627 | T |
| MTHFR C-677T | rs1801133 | T |
| MTHFR A-1298C | rs1801131 | C |
| XPC L-939G | rs2228001 | G |
| XRCC3 C18067T | rs861539 | T |
| XRCC4 G/A | rs28383151 | A |
| EGF A61G | rs4444903 | G |
| PNPLA3I148M | rs738409 | G |
| IL-28B C/T | rs12979860 | T |
| MPO G-463A | rs2333227 | A |
| HSD17B13 | rs72613567 | A |
| CAT T-262C | rs1001179 | C |
| HFE C282Y | rs1800562 | G |
| MBOAT7 | rs641738 | T |
| DEPDC5 | rs1012068 | G |
| APOE | rs429358 | T |
| CYP2R1 A/G | rs1993116 | G |
| DHCR7 T/C | rs7944926 | C |
| MICA region | rs2596542 | A |
| TM6SF2 | rs58542926 | T |
| TM6SF2 | rs187429064 | G |
| MDM2 G-309T | rs2279744 | T |
| WNT3A-WNT9A | rs708113 | T |
| P53 Arg72 Pro | rs1042522 | G |
| CD24 C170T | rs8734 | T |
| IL6 C-174G | rs1800795 | G |
| RANTES G-403A | rs2107538 | A |
| NFKβIA G-881A | rs3138053 | A |
| TLR4 G/T | rs2149356 | T |
| SOD2 A16V | rs4880 | C |
| ESR1 T29C | rs2077647 | C |

The rs number corresponds to the number of the dbSNP database maintained by the NCBI (NIH, USA and available at www.ncbi.nlm.nih.gov/snp/).

As indicated above, a gene is considered to be associated with HCC present when some alleles of the gene have been identified with a higher occurrence in patients who developed HCC, whereas other alleles have been identified with a higher occurrence with patients who didn't develop HCC.

The methods herein disclosed are particularly effective when the SNPs rs738409 (PNPLA3), rs58542926 (TM6SF2), rs187429064 (TM6SF2), rs72613567 (HSD17B13), rs429358 (APOE) and rs641738 (MBOAT7) are detected.

In a specific embodiment, SNP rs708113 in the WNT3A-WNT9A locus is also detected.

In another embodiment, the information pertaining to a combination of SNPs present in alleles specifically associated with HCC is further combined with values obtained from other markers. One can use biological markers (herein also described as biochemical markers), i.e. a marker (protein, hormone...) that is in biological media such as tissues, cells, or fluids (in particular blood, serum or plasma). In the preferred embodiment, the marker is measurable in the serum of the patient. For a biological marker, the value measured is the amount (or concentration) of the marker, potentially normalized.

One can also use clinical markers that relate to the clinical condition of the patient. For these markers, it is envisaged to assign them a discrete value (either binary 0/1, or not) depending on the patient's clinical condition at the time the marker is evaluated. The level of fibrosis or of cirrhosis may thus be graded and used in combination with the allele information and other markers.

One can also use physical markers such as sex (0 for female; 1 for male), age, diabetes status (0 for absence of diabetes, 1 for presence of diabetes), height, weight (generally Body Mass Index) of the patient.

In this embodiment, the invention thus also relates to a method for determining whether a patient has an increased risk of developing HCC, comprising:

a) providing the values of the concentration of at least two biochemical markers in the blood, serum or plasma of the patient

b) determining the presence or absence of alleles specifically associated with HCC in the genome of the patient in genes associated with HCC, wherein the alleles of at least two genes are determined, allocating a numerical value depending on the presence of none, one or two alleles specifically associated with HCC in the genome of the patient, and combining the numerical values through a mathematical function for each gene to obtain an intermediate value

c) combining the values of a) and the numerical values or the intermediate value of b) in a mathematical function, so as to obtain an end value.

As indicated above, the method is performed ex *vivo*. The values of at least two biochemical markers are used. In another embodiment, the values of at least three biochemical markers are used.

In an embodiment, the end value is compared to predetermined values (which are cutoffs or thresholds), which makes it possible to determine whether the patient has an increased risk, a lower risk or an average risk of developing an HCC.

The mathematical function of c) can be set up so as to provide the nature of the risk for a given period of time, as explained below.

In a preferred embodiment, the patient has cirrhosis, in particular non-viral cirrhosis. Should the patient have has viral cirrhosis in the past, such cirrhosis is either cured or under control (no viral nucleic acid can be detected in samples of the patient).

It is reminded that the risk is increased (or lowered) as compared to the general risk of a population of comparable patients, i.e. patients having the same clinical condition (cirrhosis, in particular non-viral, as described above).

It is preferred when the function of b) is an algebraic sum. However, other type of functions could be used

In a preferred embodiment, the function of c) also combines the values of the sex (0 for female; 1 for male) and the age (years) of the patient.

One can use any relevant biochemical markers, such as markers indicated a varying when a patient has a liver condition. In particular, the biochemical markers may be selected from $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, $\gamma$-globulin, albumin, $\alpha$1-globulin, $\alpha$2-globulin, $\beta$-globulin, IL10, TGF-$\beta$1, apoA2, apoB, cytokeratin 18, platelets number (platelet count), prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructosebisphosphate aldolase B, fumarylacetoacetase, transthyretin, PR02275, Creactive protein (isoform 1), leucine-rich alpha-2-glycoprotein, serpin A11, DNA-directed RNA polymerase I subunit RPA1, obscurin (isoform 1), alphaskeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1, carbohydrate deficient transferrin, $\alpha$-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), $\alpha$-Lfucosidase (AFU), Des-$\gamma$-carboxyprothrombin (DCP or PIVKA), Osteopontin (OPN), and Human Carbonyl Reductase.

Biochemical markers associated with a liver condition are preferably selected from the group consisting of $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, platelet count, albuminemia (serum albumin).

Biochemical markers that can be relevant for the presence of cancer, in particular liver cancer can be used, such as $\alpha$-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), $\alpha$-Lfucosidase (AFU), Des-$\gamma$-carboxyprothrombin (DCP or PIVKA), Osteopontin (OPN), or Human Carbonyl Reductase.

In a preferred embodiment, the markers are platelet count, GGT and albuminemia.

The function of c) that combines the values of biochemical markers, the intermediate value linked to the presence/absence of alleles specifically associated with HCC can be obtained by

a) providing the concentration of biochemical markers as measured in the blood, serum or plasma of patients of a cohort of patients;
b) determining the presence or absence of alleles specifically associated with HCC in the genome of the patient in genes associated with HCC, wherein the alleles of at least two genes are determined, allocating a numerical value depending on the presence of none, one or two alleles specifically associated with HCC in the genome of the patient, and combining the numerical values through a mathematical function for each gene to obtain an intermediate value
c) performing a follow-up of the patients of the cohort at the given period of time;
d) determining the occurrence of HCC for each patient of the cohort during the given period of time;
e) classifying the patients of the cohort in different groups according to the occurrence of HCC during the given period of time, and optionally the time where HCC was diagnosed;
f) identifying biochemical markers which differ significantly between these groups by unidimensional analysis;
g) performing a regression analysis to assess the independent discriminative value of the markers for the occurrence of HCC cancer during the given period of time;
h) obtaining the function by combination of the independent markers identified in g) and of the numerical value obtained in b), and optionally of age, sex, diabetes status, and/or Body Mass Index of the patient.

In one embodiment, the function is obtained by Fine-Gray regression modelling accounting for the competing risk of non-HCC related death. The Fine-Gray subdistribution hazard model estimates the effect of a set of covariates on the cumulative incidence function (CIF) of a given event of interest (here HCC), which describes the incidence of occurrence of the event while accounting for competing risks. The Fine-Gray model relies on the following formula:

$$1-\text{CIF}(t)= \left(1-\text{CIF}_0(t)\right)^{\exp(X\beta)}$$

where $\text{CIF}_0$ denotes the baseline CIF, X a set of covariates and $\beta$ denotes the vector of regression coefficients from the Fine-Gray model.

In another embodiment, the function is obtained by logistic regression modelling.

It thus appears that the predictive value of the function is appropriate for the given period of time during which the follow-up is performed. Various functions may thus be designed for various periods of time. In particular, the given period of time may be five years. The given period of year may be four years, or three years or two years.

In another embodiment, the given period of time is one year. This is of particular interest as it allows providing a specific follow-up or treatment to the patients identified as having an increased risk during the period between two routine exams.

In particular, and to obtain an evaluation of the risk at one, three and five years, one can use the following formula, derived from the Fine-Gray regression model comprising the 7 SNPs-GRS score, as a function:

Score = a1 x age (years) + a2 x Sex  + a3 x Diabetes  – a4 x platelet counts $(10^3/mm^3)$ + a5 x GGT (xN) – a6 x serum albumin (g/L) + a7 x $7SNPsGRS_7$ + a8 x $7SNPsGRS_{8-13}$

with

- $0.038 \leq a1 \leq 0.045$, preferably $0.040 \leq a1 \leq 0.042$
- $0.92 \leq a2 \leq 1.02$, preferably $0.94 \leq a2 \leq 1.00$
- $0.45 \leq a3 \leq 0.55$, preferably $0.48 \leq a3 \leq 0.52$
- $0.007 \leq a4 \leq 0.009$, preferably $0.075 \leq a4 \leq 0.080$
- $0.005 \leq a5 \leq 0.015$, preferably $0.008 \leq a5 \leq 0.013$
- $0.040 \leq a6 \leq 0.053$, preferably $0.043 \leq a6 \leq 0.050$
- $0.40 \leq a7 \leq 0.60$, preferably $0.45 \leq a7 \leq 0.55$
- $0.68 \leq a8 \leq 0.82$, preferably $0.72 \leq a8 \leq 0.78$

A specific function of interest is represented by:

$$Score = 0.04085 * Age_{years} + 0.97209 * Male\ gender + 0.50060 * Diabetes$$
$$- 0.0078 * Platelet\ count_{10^3/mm^3} + 0.01167 * GGT_{xN}$$
$$- 0.04688 * Serum\ albumin_{g/L} + 0.50834 * 7SNPsGRS_{class\ 1}$$
$$+ 0.75184 * 7SNPsGRS_{class\ 2}$$

It can also be written as

$$Score = 0.04085 * Age_{years} + 0.97209 * Male\ gender + 0.50060 * Diabetes$$
$$- 0.0078 * Platelet\ count_{10^3/mm^3} + 0.01167 * GGT_{xN}$$
$$- 0.04688 * Serum\ albumin_{g/L} + 0.50834 * 7SNPsGRS_7$$
$$+ 0.75184 * 7SNPsGRS_{8-13}$$

where Male gender, Diabetes equal to 1 when present, and to 0 otherwise (for female of no diabetes); $7SNPsGRS_7$ ($7SNPsGRS_{class1}$) equals to 1 when the sum of 7 alleles scores equals to 7, and to 0 otherwise; and $7SNPsGRS_{8-13}$ ($7SNPsGRS_{class2}$) equals to 1 when the sum of 7 alleles is between 8 and 13, and to 0 otherwise, wherein the GGT is relative to normal (xN = "x times normal"). The amount of GGT is generally measured in UI/l. However, since some variability may exist between different laboratories, and since the amount of GGT may widely vary, it is generally clinically expressed as a multiple to the upper limit of normal (ULN) of the range of the laboratory performing the measure. In the above functions, GGT is thus expressed as the multiple (x times) of the lab upper limit normal (N). This manner of expressing GGT levels is widely used clinically.

The SNPs used are rs738409 (PNPLA3), rs58542926 (TM6SF2), rs187429064 (TM6SF2), rs72613567 (HSD17B13), rs429358 (APOE), rs641738 (MBOAT7) and rs708113 (WNT3A-WNT9A locus).

The resulting calculated score can then be compared to the following reference values for the observed risk (CIF) of HCC shown in Table 2:

Table 2. Observed cumulative incidence function according to the score value

|  | Observed cumulative incidence function | | |
|---|---|---|---|
|  | **1-year follow-up** | **3-year follow-up** | **5-year follow-up** |
| *Score value* |  |  |  |
| < 0.5826 | 0.2% | 1.3% | 3.0% |
| [0.5826 and 1.1704] | 2.7% | 7.4% | 11.1% |
| > 1.1704 | 4.1% | 16.9% | 24.2% |

Consequently, using the above threshold, it is possible to identify patients with an increased risk of HCC within the chosen period of time. In this case, patients for which the end value obtained from the score described above is higher than 1.1704 (or 1.17) shall be proposed further exams than regular US exams. Patients with a score below 0.5826 (or 0.58) are considered as at a decreased risk. Patient with an end value between 0.5826 and 1.1704 have an average risk. Patients with a decreased or average risk are a *priori* not proposed other exams than UV.

This formula relies on the multivariate Fine-Gray model showed in Table 3, where adjusted regression coefficients (i.e. log(adjusted subhazard ratio [SHR])) are used as weights for the score calculation.

Table 3. Multivariate analysis using routine clinical and biological variables, and the 7 SNPs-GRS score.

| | Routine basis model + 7 SNPs-GRS | | |
| --- | --- | --- | --- |
| | Adjusted regression coefficient | Adjusted SHR [95% CI] | P-value |
| Age (years) | 0.04085 | 1.04 [1.02 ; 1.07] | **0.001** |
| Male gender | 0.97209 | 2.64 [1.55 ; 4.51] | **<0.001** |
| Diabetes | 0.50060 | 1.65 [1.04 ; 2.61] | **0.032** |
| Platelet counts ($10^3$/mm$^3$) | -0.00786 | 0.992 [0.988 ; 0.997] | **0.001** |
| GGT x $N$ (N=45) | 0.01167 | 1.012 [1.004 ; 1.020] | **0.005** |
| Serum albumin (g/L) | -0.04688 | 0.95 [0.91 ; 0.99] | **0.04** |
| 7 SNPs-GRS | | | **0.012** |
| 0-6 | | Ref | |
| 7 | 0.50834 | 1.66 [0.96 ; 2.88] | 0.07 |
| 8-13 0.75184 | | 2.12 [1.28 ; 3.52] | **0.004** |

It is to be noted that the methods herein disclosed, in particular the combination of the values and the comparison with the predetermined values, are preferably implemented by a computer.

Other functions can be determined, using other biological, clinical or physical markers, as well as other SNPs (more SNPs or different SNPs). In particular, SNP rs708113 in the WNT3A-WNT9A locus may be omitted.

Specific interesting biological markers are $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, platelet count, albuminemia (serum albumin). AFP can also optionally be used.

The invention also related to a method for determining whether a patient has an increased risk of developing HCC, comprising

a) requiring a sender to identify himself within a public or private network,

b) requiring the sender to fill in information related to the patient, and assigning a specific identifier to the patient in a database;

c) receiving values of the concentration of biochemical markers in the blood, serum or plasma of the patient, optionally as well as age, gender and diabetes status of the patients, wherein the sender is in a remote place and wherein the values are received in a secure manner, and storing the values in the database, associated with the specific identifier of the patient

d) receiving information relating to presence or absence of alleles associated with an increased risk of occurrence of hepatocarcinoma cancer in the genome of the patient, wherein the information is received in a secure manner, and storing the information in the database, associated with the specific identifier of the patient

e) allocating a numerical value for each allele, depending on the presence of none, one or two alleles associated with an increased risk of occurrence of hepatocarcinoma cancer, and storing the numerical values in the database, associated with the specific identifier of thepatient

f) combining the values of b) and the values of f) in a function to obtain an end value

g) storing the end value in a database associated with the specific identifier, thereby obtaining a database where the information related to the patient is present under the specific identifier, and preferably the date and time of identification of the sender

h) comparing the end value to a predetermined value, wherein the patient has an increased risk to have an increased risk of having a HCC in the given period of time if the end value is higher than the predetermined value, thereby obtaining a nature of the risk of occurrence of HCC,

i) sending the nature of the risk to the sender.

As for the methods described above, the patient has preferably cirrhosis, and the risk is increased as compared to the average risk of a population of patients having the same clinical conditions.

In a first step, a sender is required to identify himself to a server within a public or private network. It is preferred when the network is a private network, and when the connection to the server is encrypted. Identification of the sender can be performed using login and password, preferably through strong identification processes (such as one-time password, or using biometric or smart card identification).

Upon login to the server, the sender is required to fill-in information related to the patient (such as name, sex, birth date, height, weight, social security identification number) and a a specific identifier is assigned to the patient in a database. If the database doesn't contain any entry relating to the patient, one entry is created. If an entry already exists (using the social security identification number allows ensuring the unicity of entry), such entry is activated.

The server shall then receive values of the concentration of biochemical markers as disclosed herein, and store them in a database, associated with the specific identifier of the patient, so that such data is uniquely associated to the patient. Exchanges between the sender's device and the server are very advantageously performed in a secure manner (encrypted exhanges). Age and sex of the patient may also optionally be sent or may be calculated using the information related to the patient. The server shall then receive information relating to the presence or absence of alleles specifically associated with HCC in the genome of the patient for at least two genes associated with HCC. Such information is also preferably received in a secure manner, and stored in the database, associated with the specific identifier of the patient.

It is to be noted that the sender is generally in a remote place different from where the server is located. It is also envisaged that the information is recovered by the server, using information provided by the sender. For instance, the server may open a connection with the server of the laboratory having measured the biochemical markers and/or determiner the presence or absence of the alleles specific for HCC, using identifiers provided by the sender, to upload the required information.

The next step is for a processor to allocate, for each gene, a numerical value depending on the presence of none, one or two alleles specifically associated with HCC in the genome of the patient. Such numerical values are stored in the database, associated with the specific identifier of the patient. If the genetic information of the patient is already know, the steps of receiving the information pertaining to the alleles of the genes associated with HCC, of allocating the numerical values to these alleles can be omitted.

The following step is to combining the values relating the biochemical markers, and the values relating to the genetic information, stored in the database, and optionally the age, sex, diabetes status and/or BMI of the patient, through a function to obtain an end value

The end value is then stored in a database associated with the identifier specific of the patient and is preferably date and time-stamped. Indeed, since the method is to be performed on a regular basis, it may be interested to keep a record of the results obtained overtime. Thereby, a database is obtained, containing identification related to the patient (patient's specific identifier), associated with the biochemical and genetic data, and numerical values, including the end value as calculated.

The end value is the compared to a predetermined value, and the nature of the risk of the patient (increased risk, lower risk or average risk) of developing an HCC is determined.

Such information (nature of the risk of the patient) is then transmitted to the sender.

The information pertaining to the risk of the patient can be used for refining or improving early HCC detection in the patient, by performing the methods herein disclosed and performing screening by scanner, MRI or early detection of circulating biomarkers (including liquid biopsy) if the patient is found to have an increased risk of developing HCC, as determined by the methods herein disclosed.

Early treatment of a patient comprising administration of anti-cancer drugs, but also screening by scanner, MRI (in particular injected MRI), liver biopsy , can also be performed if the patient is found to have an increased risk of developing HCC, as determined by the methods herein disclosed..

FIGURES

Figure 1: Flow chart for the selection of patients.
Figure 2: HCC incidence as a function of genetic risk scores in the CIRRAL cohort.

A. Six SNP GRS (PNPLA3, TM6SF2, HSD17B13, MBOAT7, APOE, TM6SF2-2).
B. Seven SNP GRS (PNPLA3, TM6SF2, HSD17B13, MBOAT7, APOE, TM6SF2-2, WNT3A-WNT9A)

Figure 3: Performance of HCC prediction models
Figure 4: HCC incidence as a function of genetic risk scores (GRS) in the CIRRAL cohort. A. Six SNP GRS (PNPLA3, TM6SF2 rs58542926 and rs187429064, HSD17B3, APOE, MBOAT7). B. Seven SNP GRS (PNPLA3, TM6SF2 rs58542926 and rs187429064, HSD17B13, APOE, MBOAT7, WNT3A-WNT9A)

Figure 5: HCC incidence as a function of genetic risk scores (GRS) in the CirVir cohort. A. Six SNP GRS (PNPLA3, TM6SF2 rs58542926 and rs187429064, HSD17B3, APOE, MBOAT7). B. Seven SNP GRS (PNPLA3, TM6SF2 rs58542926 and rs187429064, HSD17B13, APOE, MBOAT7, WNT3A-WNT9A)

Figure 6: Non-HCC mortality as a function of genetic risk scores (GRS) in the whole cohort. A. Six SNP GRS (PNPLA3, TM6SF2 rs58542926 and rs187429064, HSD17B3, APOE, MBOAT7). B. Seven SNP GRS (PNPLA3, TM6SF2 rs58542926 and rs187429064, HSD17B13, APOE, MBOAT7, WNT3A-WNT9A)

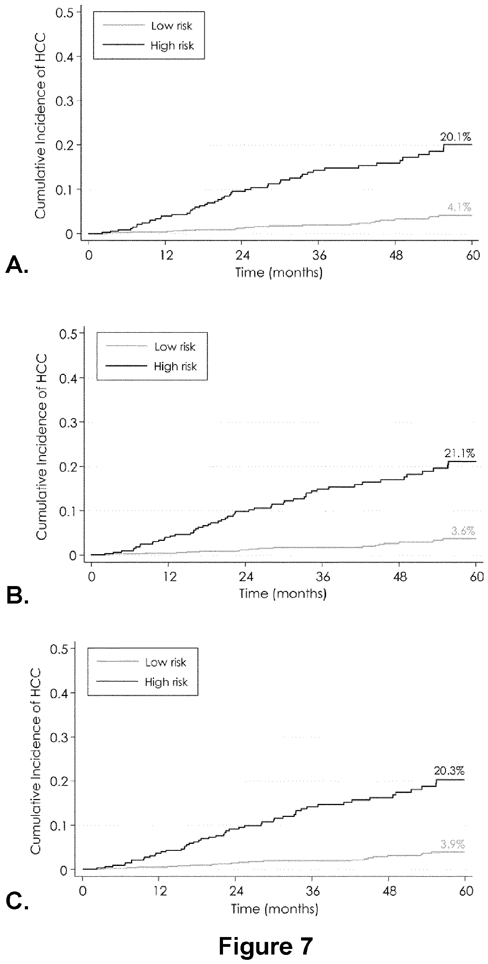

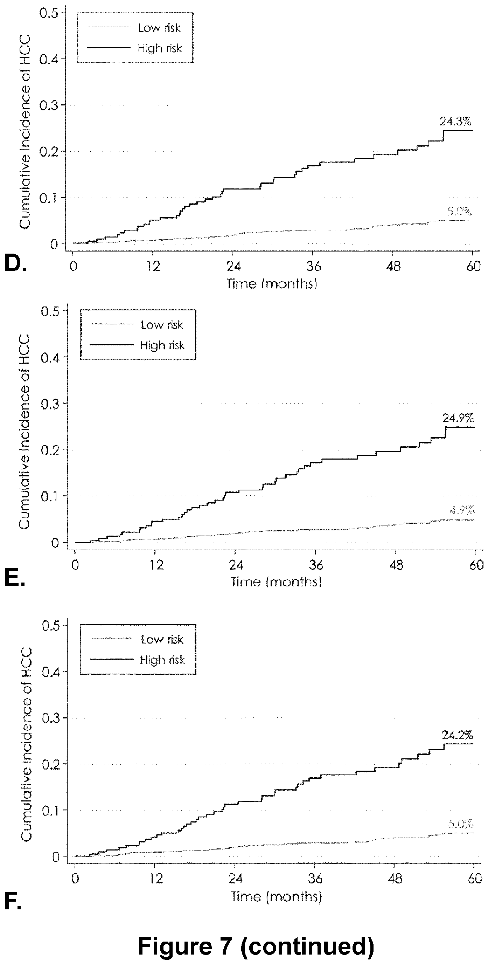

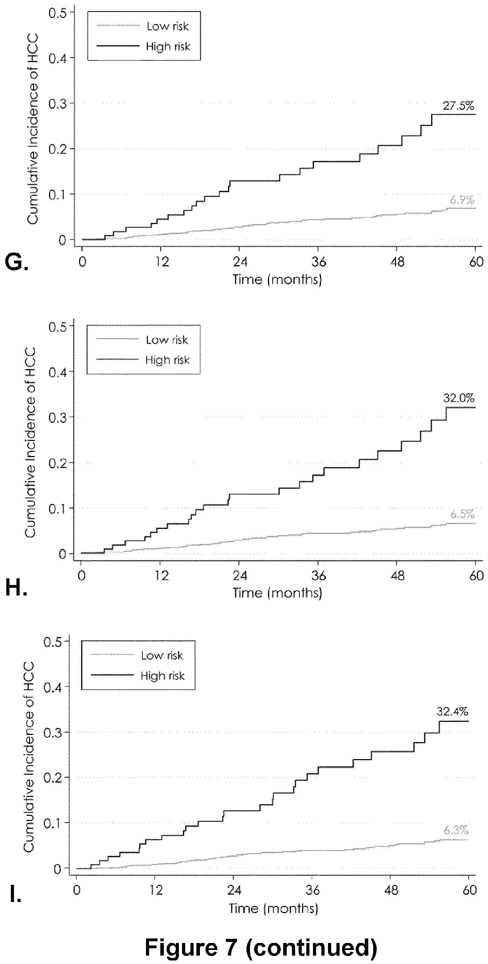

Figure 7: comparison of cumulative incidence of 5-yrs HCC for high/low-risk patients, defined by clinical model alone (A, D, G) vs clinical model+6-SNPs GRS (B, E, H) and clinical model+7-SNPs GRS (C, F, I). High/low-risk was defined according to illustrative percentile cut-off points (A, B, C high risk definition >70th percentile; D, E, F high risk definition >80th percentile; G, H, I high risk definition >90th percentile). As an example, the 70th percentile definition means that individuals whose score was in the 70th percentile or greater (i.e. in the top 30%) were categorized as high risk, and the remainder were categorized as low risk. GRS, genetic risk score.

Figure 8: Comparison of cumulative incidence of HCC at 5 years for high/low-risk patients, defined by aMAP score (A, D, G) vs aMAP score combined with GRS (6 SNP (B, E, H) or 7 SNP (C, F, I)). High/low-risk was defined according to illustrative percentile cut-off points (A, B, C high risk definition >70th percentile; D, E, F high risk definition >80th percentile; G, H, I high risk definition >90th percentile).

## EXAMPLES

## Example 1. Patients And Methods

### *Patients*

Data from two French prospective cohorts of patients with biopsy-proven compensated cirrhosis without detectable focal liver lesions at inclusion were used. These cohorts have already been extensively described: the ANRS CO12 CirVir [20] and CIRRAL cohorts [21]. Each study was conducted in accordance with the ethical guidelines of the 1975 Declaration of Helsinki and French laws for biomedical research and was approved by the ethics committees. They are both reported according to the STROBE Statement. All patients gave written informed consent to participate. None of the patients from these cohorts were previously included in the previously published French GWAs [12]; conversely to the CIRRAL or CirVir cohorts which considered patients regularly screened for HCC and in whom follow-up was monitored according to pre-defined protocols, this two-stage case-control GWAS only selected patients who were referred for chronic liver disease and/or HCC management and do not comprise any recorded longitudinal follow-up for research purposes.

All patients enrolled in these cohorts had periodic liver ultrasonographic (US) surveillance according to international and French guidelines, with or without AFP serum level dosage. In the case of detected focal liver lesions, a recalled diagnostic procedure using contrast-enhanced imaging (computed tomography scan or MRI) and/or guided biopsy was performed according to the 2005 AASLD guidelines updated in 2011 [22, 23]. A diagnosis of HCC was thus established by either histological examination or based on probabilistic non-invasive criteria (mainly dynamic imaging revealing early arterial hyperenhancement and washout on portal venous or delayed phases)) according to the different time periods (before and after 2011). When HCC diagnosis was established, treatment was determined using a multidisciplinary approach according to AASLD [22, 23] and the EASL-EORTC [24] guidelines.

In addition to HCC occurrence [25, 26], which was the primary endpoint of both cohorts, all events that occurred during follow-up (i.e., death, liver decompensation, bacterial infection [25], extrahepatic malignancies [27] and cardiovascular diseases [28]) were recorded using information obtained from the medical records of patients held by each centre [29]. Moreover, likely cause(s) of death were established. All recorded information during follow-up was secondarily monitored by clinical research associates localized in institution 1, 3 and 7. All medical diagnoses of events occurring during follow-up were confirmed by two senior hepatologists (authors N.G-C and P.N.).

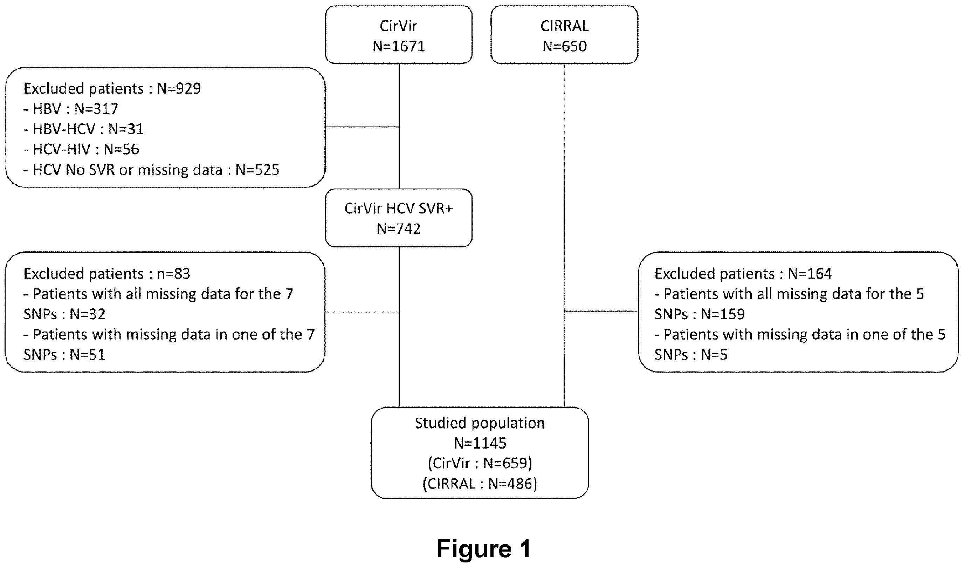

Patients who underwent liver transplantation were censored for analysis at the date of transplantation. All treatments, including antiviral therapies, were recorded at inclusion, and patients were notified of any modifications during follow-up [30]. A single database encompassing clinical data from the 2 cohorts was built on November 18, 2019 [29]. Among all included patients, only those with alcoholic cirrhosis or who achieved HCV eradication during follow-up were considered for the present analyses, the date of viral eradication being set as index time (see Figure 1).

### *ANRS CO12 CirVir cohort*

The ANRS CO12 CirVir cohort, sponsored and funded by the ANRS (France REcherche Nord & Sud Sida-HIV Hépatites), is a multicentre observational cohort that aims to characterize the incidence of complications occurring in biopsy-proven compensated cirrhosis and to identify the associated risk factors using competing risks analysis [20]. The full CirVir protocol is available on the ANRS website (anrs.fr). Specific additional inclusion criteria were i) cause of cirrhosis related to either chronic infection with HCV and/or HBV regardless of the levels of replication and alcohol consumption, ii) patients belonging to Child-Pugh A at enrolment, iii) absence of previous hepatic complications (particularly ascites, gastrointestinal haemorrhage, or HCC), and iv) absence of severe uncontrolled extrahepatic disease resulting in an estimated life expectancy of less than 1 year.

Among the 1822 patients recruited in 35 French clinical centres between March 2006 and July 2012, 151 were subsequently excluded from analysis after reviewing individual data due to either noncompliance with inclusion criteria (n = 142) or consent withdrawal (n = 9), leading to a total of 1671 patients selected for further analysis, including the present one.

*CIRRAL cohort*

CIRRAL is a multicentre cohort study implemented in 22 French and 2 Belgian tertiary liver centres to capture the whole spectrum of complications occurring in compensated alcoholic cirrhosis using competing risk analyses [21]. The promoter was APHP. The cohort was funded by the French National Institute of Cancer (INCa), the French Association for Research in Cancer and the ANRS (PAIR CHC 2009) and was registered on ClinicalTrials.gov (NCT00190385). Specific additional inclusion criteria were i) cause of cirrhosis related to chronic alcohol abuse according to the World Health Organization criteria (more than 21 glasses per week for females and more than 28 glasses per week for males) for at least 10 years, ii) absence of chronic infection with HCV or HBV, and iii) patients belonging to Child-Pugh A at enrolment. The follow-up of patients was strictly superposed on the ANRS CO12 Cirvir cohort design.

Among the 706 patients included between October 2010 and April 2016, 54 were subsequently excluded after reviewing individual data because of violations of the inclusion criteria (n = 48) or consent withdrawal (n = 6); ultimately, 652 patients were selected for further analysis, including the present one.

*DNA storage, extraction and genotyping*

DNA samples were prepared from blood samples collected in all participating centres and then centralized by the liver biobank of the Plateforme de Ressources Biologiques des Hôpitaux Universitaires Paris Seine-Saint-Denis (BB-0033-00027), Assistance-Publique Hôpitaux de Paris, Bobigny, France. All patients gave written consent for blood sampling and genotyping. This study was approved by the Comité de Protection des Personnes d'Aulnay-sous-Bois, France. Genomic DNA was extracted from each patient's peripheral blood mononuclear cells using a MagNA Pure Compact Instrument (Roche Diagnostics).

Patients were genotyped for rs738409 (PNPLA3 I148M variant), rs58542926 (TM6SF2 E167K), rs187429064 (TM6SF2), rs641738 (C>T MBOAT7), rs72613567 (HSD17B13:TA), rs429358 (APOE) and rs708113 (WNT3A-WNT9A). MBOAT7, TM6SF2, WNT3A-WNT9A and PNPLA3 SNPs were genotyped by allelic discrimination using fluorogenic probes and appropriate TaqMan assays (rs641738: C_8716820_10; rs738409: C_7241_10; rs58542926: C_89463510_10; rs708113: C_11576791_10, Thermo Fisher). HSD17B13 rs72613567 genotyping was performed using custom primers and probes (forward primer: GCT CTA TTG GTG TTT TAG TAT TTG GGT GTT (SEQ ID NO: 1), reverse primer: TGT TCC ATC GTA TAT CAA TAT CTT TCT GAG ACT (SEQ ID NO: 2), qHSD17B13-A: CTG TGC TGT ACT TAC TTC T (SEQ ID NO: 3), qHSD17B13-AA: TGC TGT ACT TAA CTT CT (SEQ ID NO: 4).

PCRs (25 μl) consisted of 1x TaqMan Universal PCR master mix (Applied Biosystems), 1X assay mix, and 10 ng of genomic DNA. Real-time PCR was carried out on a Step One Plus PCR system (Applied Biosystems) using a protocol consisting of incubation at 50 °C for 2 minutes and 95 °C for 10 minutes, followed by 40 cycles of denaturation at 92 °C for 15 seconds and annealing/extension at 60 °C for 1 minute. The FAM and VIC fluorescence levels of the PCR products were measured at 60 °C for 1 minute, resulting in the clear identification of all genotypes of each SNP on a two-dimensional graph.

Ethnicity was defined by a predictive panel of 26 SNPs assessed on peripheral DNA. Samples were classified as European, Sub-Sahara African, or East-Asian based on the closest 1000 Genomes population in a principal component analysis [31].

*Statistical analyses*

The baseline was defined as the date of inclusion in the corresponding cohort for patients with alcoholic cirrhosis and the date of SVR achievement for patients with HCV-related cirrhosis.

Descriptive results are presented as medians (interquartile range [IQR]) for continuous variables and as numbers (percentages) for categorical data. The characteristics of patients at the baseline date were compared between the two subsets of the cohort using t tests or Mann-Whitney rank-sum tests for continuous variables and the chi-squared test or Fisher's exact test for categorical variables. The cumulative incidence of HCC was estimated in a competing risk framework, considering non-HCC death as a competing event. Unadjusted comparisons of incidence curves were performed using the Gray test. Fine-Gray regression modelling was used to determine independent baseline features associated with HCC occurrence to compute subhazard ratios (SHRs) along with their 95% confidence intervals [95% CIs]. To do so, non-SNPs clinical and routine biological variables associated with HCC risk at the P<0.20 level in univariate analysis were entered in multivariate analysis, and we applied a backwards stepwise approach to retain significant factors at the P<0.05 level until reaching a final model, thereafter called the "routine basis model". The combined influence of the studied SNPs was analysed in the whole population by creating specific GRSs coding as 0, 1, and 2 for noncarriers and heterozygous and homozygous carriers of the HCC risk-increasing allele of each variant, respectively. Then, the GRSs were added to the routine basis model to assess their independent contribution to HCC prediction, in addition to the clinical features. The same method was secondarily applied with an external HCC clinical score applicable to patients with ACLD regardless of its cause, the aMAP score, encompassing older age, male sex, albumin-bilirubin and platelets [19].

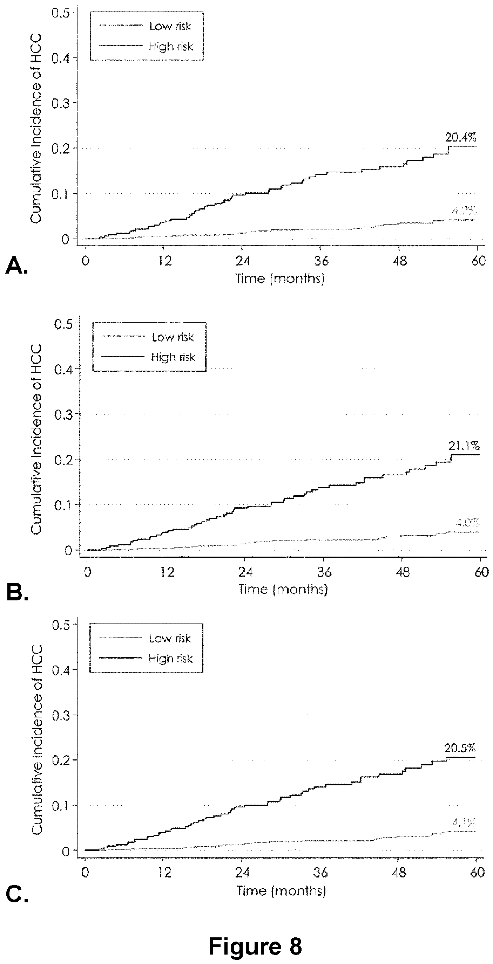

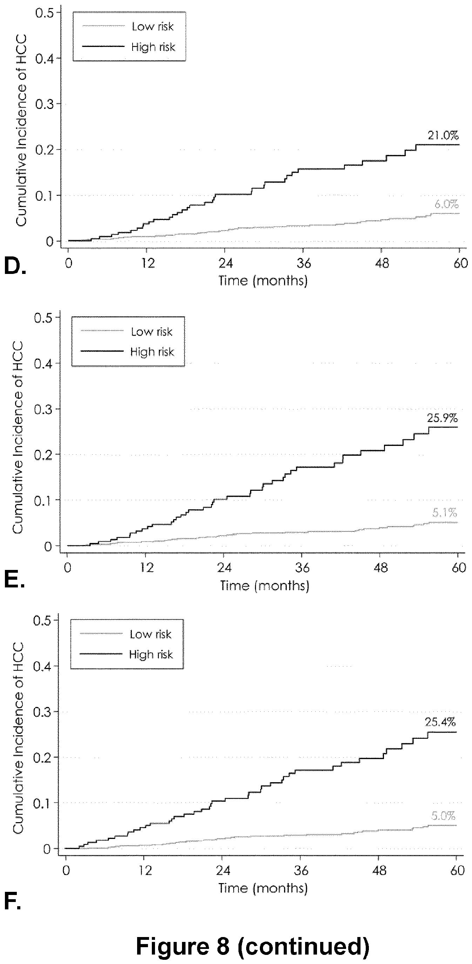

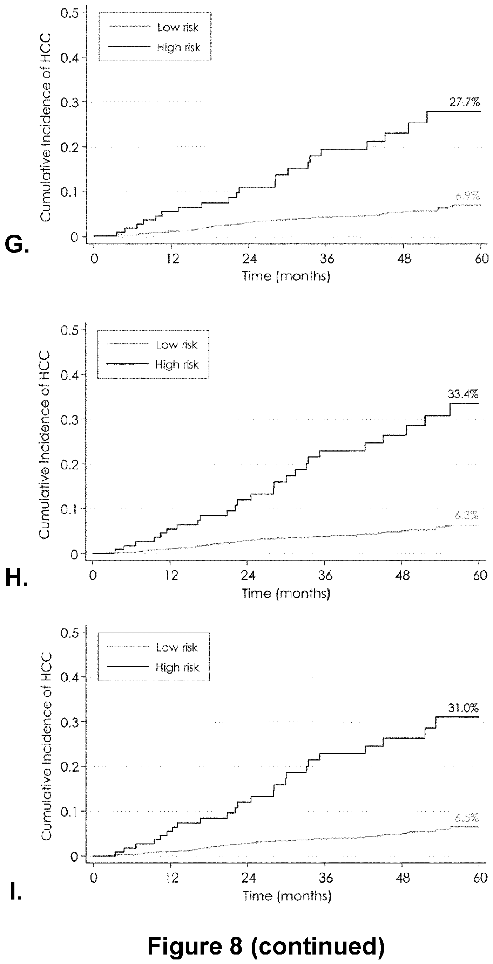

The prognostic value of the models (without and with adjustment with SNP parameters) was assessed through three approaches. First, the Wolber's concordance index (C-index) for prognostic models with competing risks was calculated [32]. Second, two risk groups "high" and "low" were created by dichotomising the predictive risk score from each multivariate model at four cut-off points: a) 70th percentile; b) 80th percentile; c) 90th percentile; and d) 95th percentile. The 5 year cumulative incidence of HCC was calculated in each of the created two groups (Figures 7 and 8, not showing the 95th percentile). The more discriminative predictive risk score, the more the cumulative incidence of the two groups are separated. Third, we estimated the standardized 'net benefit' derived from decision curve analysis [33, 34]. In summary, decision curve analysis is an increasingly used method for evaluating alternative diagnostic or prognostic strategies, helping to identify the one with the highest clinical utility or 'net benefit' (shown as the highest plotted curve). Net benefit represents the proportion of patients with true positive results minus the proportion with false-positives multiplied by the risk of HCC at each risk threshold considered. This approach thus incorporates the consequences of the decisions made based on the prognostic model.

Unadjusted analyses were conducted on complete cases without missing information, while imputation using the random forests with the R package missForest was performed in all multivariate Fine-Gray regression analyses. Statistical analyses were performed using Stata 16.0 (StataCorp, College Station, TX) and R v4.0.2 (R Foundation for Statistical Computing, Vienna, Austria). P values <0.05 were considered to be statistically significant.

## Example 2. Results

*Selection, baseline characteristics of patients and genotyping results*

A total of 2321 patients with compensated cirrhosis that underwent HCC surveillance and included in the 2 cohorts were considered (see flow chart, Figure 1). Among them, 1176 were excluded, mostly because of HBV or HIV infection or persistent HCV viral infection during follow-up or missing data for SNPs. As in all previously published analyses conducted in the CirVir and other cohorts (ref), end of treatment was defined as time 0 for patients with SVR during follow-up evaluation because patients with undetectable HCV RNA at that time were considered to have SVR status. The remaining 1145 patients had either alcoholic and/or cured HCV infection and were included in all subsequent analyses. Their baseline characteristics and genotyping results are displayed in Table 4.

Table 4. Baseline characteristics and genotyping results. AFP, alpha-fetoprotein; ALT, alanine aminotransferase; AST, aspartate aminotransferase; BMI, body mass index; GGT, $\gamma$-glutamyltransferase; PT, prothrombin time

| | | Availa ble data | HCV-cured N=659 | Alcohol N=486 | Total N=1145 | Pvalue |
|---|---|---|---|---|---|---|
| Ancestry | | 1142 | | | | <0.001 |
| | European | | 593 (90.0) | 469 (97.1) | 1062 (93.0) | |
| | Subsahara | | 52 (7.9) | 13 (2.7) | 65 (5.7) | |
| African | | | | | | |
| | East Asian | | 14 (2.1) | 1 (0.2) | 15 (1.3) | |
| | Age (years) | 1145 | 57 [51 - 65] | 58 [52 - 64] | 58 [51 - 65] | 0.634 |
| Male gender | | 1145 | 421 (63.9) | 333 (68.5) | 754 (65.9) | 0.102 |
| | BMI (kg/m$^2$) | 906 | 25.7 [22.9 - 29.3] | 27.5 [24.1 - 30.9] | 26.5 [23.5 -30.1] | <0.001 |

(continued)

| | Availa ble data | HCV-cured N=659 | Alcohol N=486 | Total N=1145 | Pvalue |
|---|---|---|---|---|---|
| BMI (kg/m$^2$) | 906 | | | | <0.001 |
| <25 | | 179 (41.2) | 154 (32.7) | 333 (36.75) | |
| [25 ; 30[ | | 169 (38.8) | 164 (34.8) | 333 (36.75) | |
| ≥30 | | 87 (20.0) | 153 (32.5) | 240 (26.5) | |
| Diabetes | 1144 | 130 (19.7) | 110 (22.7) | 240 (21.0) | 0.225 |
| Past excessive alcohol consumption | 1109 | 191 (30.7) | 486 (100) | 677 (61.1) | <0.001 |
| Platelet counts (10$^3$/mm$^3$) | 1056 | 150.0 [99.0 - 197.0] | 142.0 [102.0 - 192.0] | 147.0 [1010.5 - 195.5] | 0.291 |
| AST (IU/L) | 1054 | 31.0 [25.0 - 42.0] | 35.0 [27.0 - 50.0] | 32.0 [25.0 - 47.0] | <0.001 |
| AST x N (N=40) | 1054 | 0.78 [0.63 - 1.05] | 0.88 [0.68 - 1.25] | 0.80 [0.63 - 1.18] | <0.001 |
| ALT (IU/L) | 1058 | 29.0 [21.0 - | 26.0 [20.0 | 27.5 [21.0 | 0.007 |
| Ancestry<br>　　　　　European<br>　　　　　Subsahara African<br>　　　　　East Asian | 1142 | 593 (90.0)<br>52 (7.9)<br><br>14 (2.1) | 469 (97.1)<br>13 (2.7)<br><br>1 (0.2) | 1062 (93.0)<br>65 (5.7)<br><br>15 (1.3) | **<0.001** |
| | | 43.0] | - 40.0] | - 42.0] | |
| ALT x N (N=40) | 1058 | 0.73 [0.53 - 1.08] | 0.65 [0.50 - 1.00] | 0.69 [0.53 - 1.05] | **0.007** |
| GGT (IU/L) | 998 | 48.0 [29.0 - 86.0] | 114.0 [54.0 - 228.0] | 66.0 [38.0 - 151.0] | **<0.001** |
| GGT x N (N=45) | 998 | 1.07 [0.64 - 1.91] | 2.53 [1.20 - 5.07] | 1.47 [0.84 - 3.36] | **<0.001** |
| PT (%) | 939 | 89.0 [79.0 - 100.0] | 78.0 [67.0 - 91.0] | 84.0 [73.0 - 96.0] | **<0.001** |
| Serum albumin (g/L) | 902 | 42.7 [39.6 - 46.0] | 40.0 [36.9 - 43.0] | 41.5 [38.0 - 44.3] | **<0.001** |
| Total bilirubin (µmol/L) | 975 | 10.0 [7.0 - 15.0] | 14.0 [9.7 - 20.5] | 12.0 [8.0 - 18.0] | **<0.001** |
| AFP (ng/mL) | 824 | 3.6 [2.4 - 5.6] | 3.8 [2.5 - 5.7] | 3.7 [2.4 - 5.6] | 0.628 |
| aMAP score | 840 | 57.2 [52.4 - 62.0] | 60.2 [54.2 - 64.7] | 58.8 [53.3 - 63.5] | **<0.001** |
| *PNPLA3* (rs738409)<br>　　　　　C :C<br>　　　　　C :G<br>　　　　　G :G | 1145 | 349 (53.0)<br>255 (38.7)<br>55 (8.3) | 217 (44.7)<br>197 (40.5)<br>72 (14.8) | 566 (49.4)<br>452 (39.5)<br>127 (11.1) | **0.001** |
| *TM6SF2* (rs58542926)<br>　　　　　C :C | 1145 | 567 (86.0) | 405 (83.3) | 972 (84.9) | **0.381** |
| Ancestry | 1142 | | | | **<0.001** |

(continued)

| | Availa ble data | HCV-cured N=659 | Alcohol N=486 | Total N=1145 | Pvalue |
|---|---|---|---|---|---|
| European | | 593 (90.0) | 469 (97.1) | 1062 (93.0) | |
| Subsahara African | | 52 (7.9) | 13 (2.7) | 65 (5.7) | |
| East Asian | | 14 (2.1) | 1 (0.2) | 15 (1.3) | |
| C :T | | 89 (13.5) | 79 (16.3) | 168 (14.7) | |
| T :T | | 3 (0.5) | 2 (0.4) | 5 (0.4) | |
| **TM6SF2 (rs187429064)** | 1145 | | | | 0.163 |
| A :A | | 647 (98.2) | 471 (96.9) | 1118 (97.6) | |
| A :G | | 12 (1.8) | 15 (3.1) | 27(2.4) | |
| **HSD17B13 (rs72613567)** | 1145 | | | | 0.108 |
| | | 408 (61.9) | 318 (65.4) | 726 (63.4) | |
| A :- | | 218 (33.1) | 155 (31.9) | 373 (32.6) | |
| A :A | | 33 (5.0) | 13 (2.7) | 46 (4.0) | |
| **APOE (rs429358)** | 1145 | | | | <0.001 |
| C :C | | 11 (1.7) | 6 (1.2) | 17 (1.5) | |
| C :T | | 102 (15.5) | 120 (24.7) | 222 (19.4) | |
| T :T | | 546 (82.8) | 360 (74.1) | 906 (79.1) | |
| **MBOAT7 (rs641738)** | 1145 | | | | 0.122 |
| C :C | | 197 (29.9) | 124 (25.5) | 321 (28.0) | |
| C :T | | 336 (51.0) | 249 (51.2) | 585 (51.1) | |
| T :T | | 126 (19.1) | 113 (23.3) | 239 (20.9) | |
| **WNT3A-WNT9A (rs708113)** | 1145 | | | | 0.491 |
| A :A | | 251 (38.1) | 188 (38.7) | 439 (38.3) | |
| Ancestry | 1142 | | | | <0.001 |
| European | | 593 (90.0) | 469 (97.1) | 1062 (93.0) | |
| Subsahara African | | 52 (7.9) | 13 (2.7) | 65 (5.7) | |
| East Asian | | 14 (2.1) | 1 (0.2) | 15 (1.3) | |
| A :T | | 322 (48.9) | 224 (46.1) | 546 (47.7) | |
| T :T | | 86 (13.0) | 74 (15.2) | 160 (14.0) | |

HCC occurrence, competing event incidence and impact of genetic variants on outcomes

After a median follow-up of 66.3 [IQR: 41.4-89.3] months, 86 (7.4%) patients developed HCC, with a corresponding 5-yr incidence of 8.8% [95% CI: 6.9-10.9]. During the same timeframe, 142 (12.4%) patients died [causes of death: HCC-related at 19 (16.2%), liver-related at 34 (29.1%), extrahepatic cause at 64 (54.7%); missing data at 25]. The 5-yr non-HCC mortality incidence was 11.4% [95% CI: 9.2-13.8]. Table 5 shows the HCC SHR for each SNP in the CirVir and CIRRAL cohorts and then on the whole population under study.

Table 5. HCC subhazards ratios. SHR, subhazard ratio

| SNP | HCV-cured N=659 | Alcohol N=486 | Total N=1145 |
|---|---|---|---|
| **PNPLA3 (rs738409)** | | | |
| C :C (reference) | SHR=1.64 [0.86 ;3.15] | SHR=1.52 [0.85 ;2.73] | SHR=1.64[1.06 ; 2.53] |
| C :G or G :G | P=0.134 | P=0.158 | **P=0.025** |
| **TM6SF2 (rs58542926)** | | | |

(continued)

| SNP | HCV-cured N=659 | Alcohol N=486 | Total N=1145 |
|---|---|---|---|
| C :C (reference)C :T or T :T | SHR=1.01 [0.39;2.59] P=0.983 | SHR=1.66 [0.86;3.19] P=0.129 | SHR=1.42[0.83 ; 2.42] *P*=0.201 |
| *TM6SF2* (rs187429064) A :A (reference) A :G | SHR=3.93 [1.10;14.04] **P=0.035** | NA | SHR=1.02[0.26 ; 4.00] P=0.979 |
| *HSD17B13* (rs72613567) - :- A :- or A :A (reference) | SHR=1.15 [0.58; 2.25] P=0.691 | SHR=1.45 [0.79; 2.66] P=0.227 | SHR=1.31 [0.84 ; 2.06] P=0.235 |
| *MBOAT7* (rs641738) C :C (reference) C :T or T :T | SHR=1.43 [0.68;3.01] P=0.345 | SHR=1.83 [0.85; 3.94] P=0.122 | SHR=1.66[0.98 ; 2.83] P=0.060 |
| *APOE* (rs429358) C :C or C:T (reference) T :T | SHR=1.54 [0.55; 4.35] P=0.414 | SHR=1.23 [0.63; 2.39] P=0.544 | SHR=1.23 [0.71 ; 2.14] P=0.464 |
| *WNT3A-WNT9A* (rs708113) A :A A :T or T :T (reference) | SHR=0.90 [0.46 ;1.77] P=0.770 | SHR=2.34 [1.33 ;4.13] *P=0.003* | SHR=1.57[1.03 ; 2.39] **P=0.037** |

Patients with at least one G-PNPLA3 allele (n=579) had a higher HCC incidence (n=53 [9.2%] with a 5-year HCC incidence of 11.3% [95% CI: 8.4-14.7]) than CC-PNPLA3 homozygotes (n=33/566 [5.8%] with a 5-year HCC incidence of 6.2% [95% CI: 4.0-9.0]); SHR=1.64 [95% CI: 1.06-2.53], P=0.025. PNPLA3 (rs738409) did not influence non-HCC liver-related mortality (SHR=1.44 [95% CI: 0.73-2.87], P=0.29).

Patients with at least one T-TM6SF2 rs58542926 allele (n=173) had a similar HCC incidence (n=17 [9.8%] with a 5-year HCC incidence of 11.2% [95% CI: 6.4-17.5]) as CC-TM6SF2 rs58542926 homozygotes (n=69/972 [7.1%] with a 5-year HCC incidence of 8.3% [95% CI: 6.3-10.7]); SHR=1.42 [95% CI: 0.83-2.42], P=0.201. TM6SF2 rs58542926 did not influence non-HCC liver-related mortality (SHR=0.18 [95% CI: 0.02-1.30], P=0.09).

Patients with at least one G-TM6SF2 rs187429064 allele (n=27) had a similar HCC incidence (n=2 [7.4%] with a 5-year HCC incidence of 3.7% [95% CI: 0.3-15.9]) as AA-TM6SF2 rs187429064 homozygotes (n=84/1118 [7.5%] with a 5-year HCC incidence of 8.9% [95% CI: 7.0-11.1]); SHR=1.02 [95% CI: 0.26-4.00], P=0.979. TM6SF2 rs187429064 did not influence non-HCC liver-related mortality (SHR=1.22 [95% CI: 0.17-9.02], P=0.85).

Patients with at least one A-HSD17B13 allele (n=419) had a similar HCC incidence (n=27 [6.4%] with a 5-year HCC incidence of 6.6% [95% CI: 4.0-10.1]) as - :-HSD17B13 homozygotes (n=59/726 [8.1%] with a 5-year HCC incidence of 10.0% (95% CI: [7.5-12.9]); SHR=0.76 [95% CI: 0.48-1.20], P=0.235. HSD17B13 (rs72613567) did not influence non-HCC liver-related mortality (SHR=1.34 [95% CI: 0.68-2.63], P=0.40).

TT-APOE homozygous patients (n=906) had a similar HCC incidence (n=71 [7.8%] with a 5-year HCC incidence of 9.6% [95% CI: 7.4-12.2]) as patients with at least one C-APOE allele (n=15/239 [6.3%] with a 5-year HCC incidence of 5.6% [95% CI: 2.8-9.7]); SHR=1.23 [95% CI: 0.71-2.14], P=0.464. APOE (rs429358) did not influence non-HCC liver-related mortality (SHR=1.15 [95% CI: 0.48-2.78], P=0.75). Patients with at least one T-MBOAT7 allele (n=824) had a similar HCC incidence (n=69 [8.4%] with a 5-year HCC incidence of 10.2% [95% CI: 7.8-13.0]) as CC-MBOAT7 homozygotes (n=17/321 [5.3%] with a 5-year HCC incidence of 5.2% [95% CI: 2.9-8.6]); SHR=1.66 [95% CI: 0.98-2.83], P=0.06. MBOAT7 (rs641738) did not influence non-HCC liver-related mortality (SHR=1.60 [95% CI: 0.69-3.70], P=0.27).

AA-WNT3A-WNT9A homozygous patients (n=439) had a higher HCC incidence (n=42 [9.6%], 5-year HCC incidence 10.3% [95% CI: 7.2-14.1]) than patients with at least one T-WNT allele (n=44/706 [6.2%], 5-year HCC incidence 7.8% [95% CI: 5.6-10.5]); SHR=1.57 [95% CI: 1.03-2.39], P=0.037. WNT (rs708113) did not influence non-HCC liver-related mortality (SHR=0.91 [95% CI: 0.45-1.83], P=0.79).

*Construction of genetic risk scores (GRSs)*

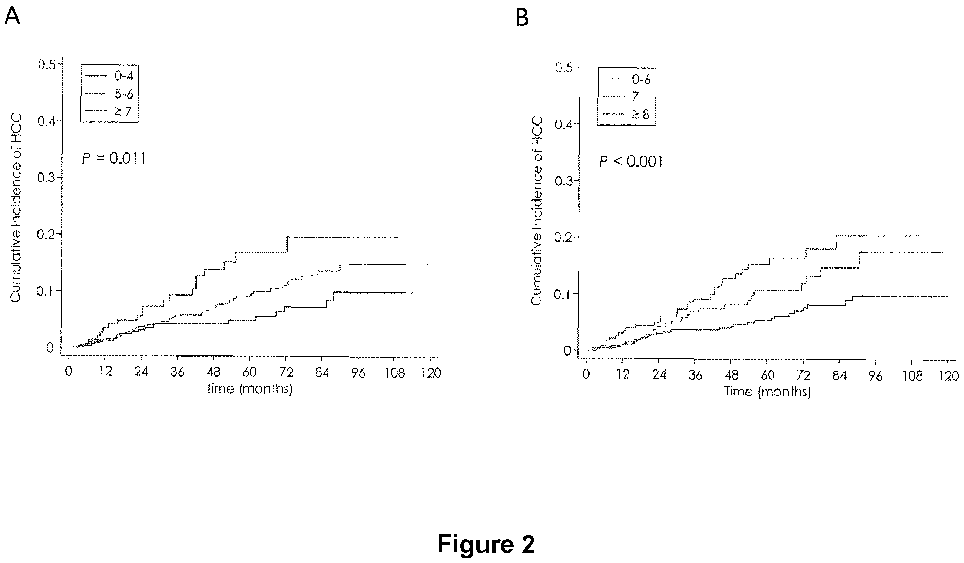

The combined influence of the six SNPs modulating liver fat content (PNPLA3, TM6SF2 rs58542926 and rs187429064, HSD17B13, APOE, and MBOAT7 genotypes) was first analyzed in the whole population by coding as 0, 1, and 2 for non-carriers, heterozygous and homozygous carriers of the HCC risk-increasing allele of each variant, respectively. In a first step, a combined 6-SNP GRS was calculated as the unweighted sum of these HCC risk-increasing alleles (range, 0-12) for each participant. Because of low numbers in some groups, subsequent analyses were conducted as a function of three genotypic associations (Group 1: scores 0-4, n=363; Group 2: scores 5-6, n=622; Group 3: scores ≥7, n=160). The 5-year HCC incidence increased progressively from Group 1 (4.8% [95% CI: 2.7-7.8]), to Group 2 (9.1% [95% CI: 6.5-12.2]), to Group 3 (16.8% [95% CI: 10.1-24.9]), Pglobal=0.011 (Figure 2A). After exclusion on non-European patients, the 6-SNP GRS remained associated with the 5-year HCC incidence (*Pglobal*=0.007)*.*

In a second step, WNT3A-WNT9A genotypes were similarly added to the previous GRS, yielding a range from 0 to 14 for the 7-SNP score. Subsequent analyses were conducted as a function of three genotypic associations (Group 1: scores 0-6, n=627; Group 2: score 7, n=276; Group 3: scores ≥8, n=242). The 5-year HCC incidence increased progressively from Group 1 (5.4% [95% CI: 3.5; 7.8]), to Group 2 (10.7% [95% CI: 6.6; 15.9]), to Group 3 (15.3% [95% CI: 10.2; 21.4]); Pglobal<0.001 (Figure 2B). After exclusion on non-European patients, the 7-SNP GRS remained associated with the 5-year HCC incidence (*Pglobal*=0.001).

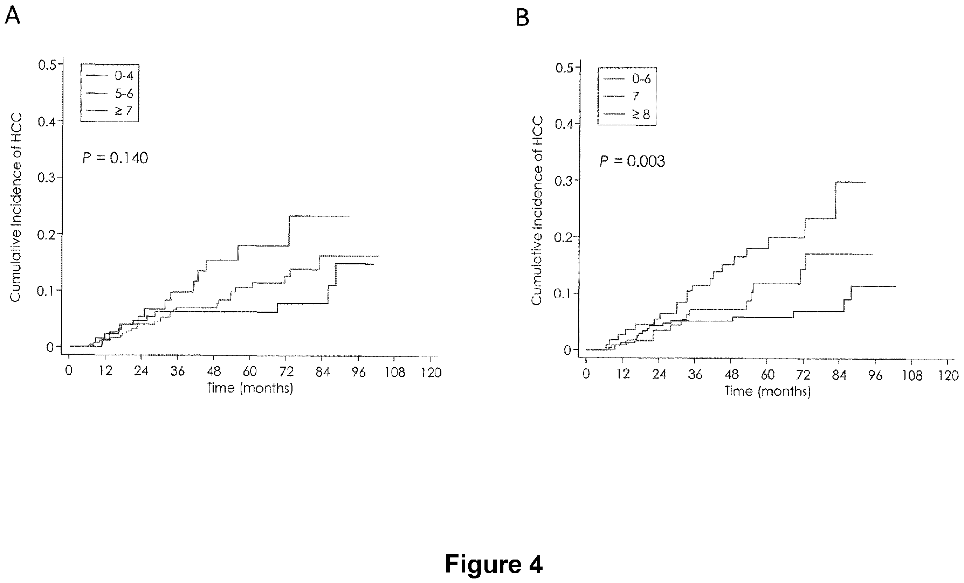

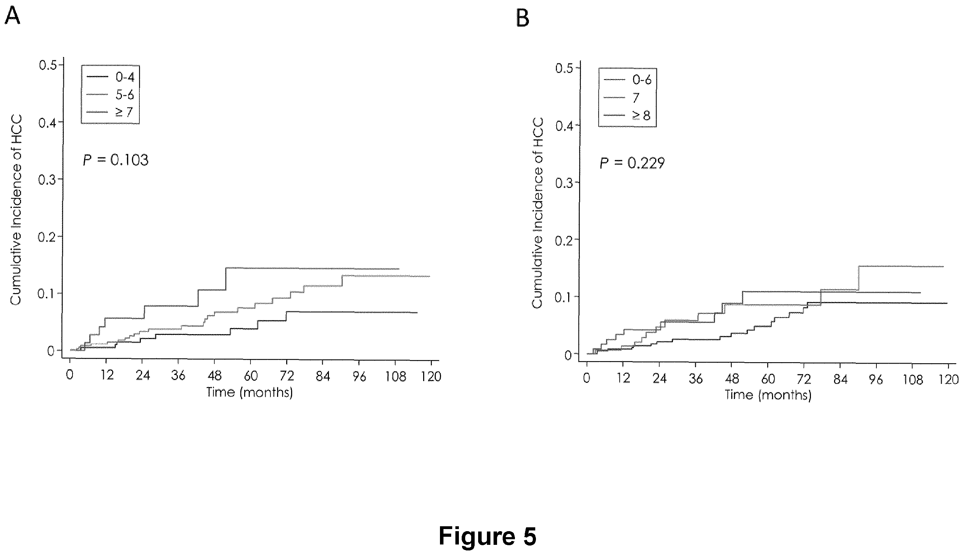

When these scoring systems were restricted to CIRRAL (Figure 4) or CirVir cohorts (Figure 5), the 7-SNP GRS was the only score significantly associated with HCC in ALD patients, while the 6-SNP score nearly reached statistical significance in both cohorts.

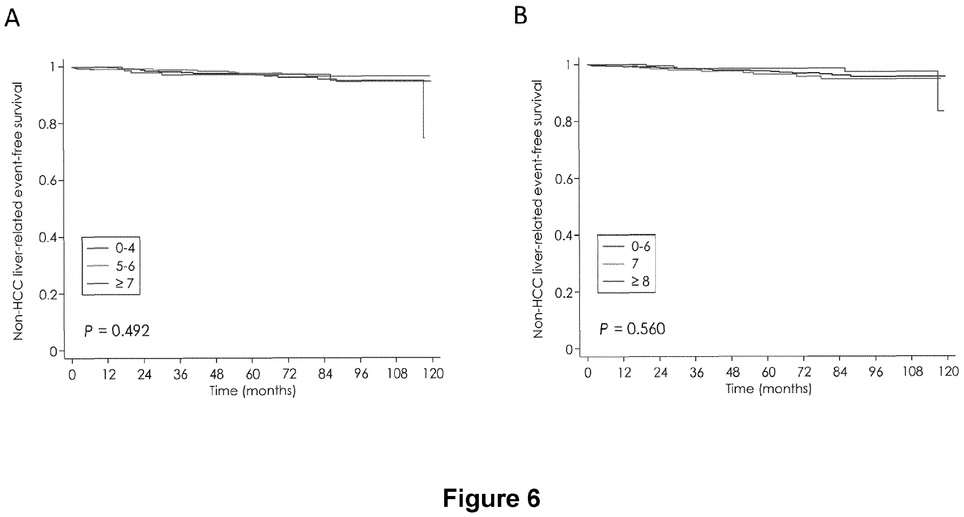

Neither the 6-SNP GRS nor the 7-SNP GRS affected non-HCC liver-related mortality (Figure 6A and 6B, respectively).

*Features associated with HCC occurrence*

Table 6 displays the results from univariate analyses using Fine-Gray regression models. The model identified several parameters as HCC risk factors taking into account competing risks of death. Similarly, the aMAP score was also associated with HCC occurrence. Multivariate analyses were subsequently performed to assess the added prognostic value of the 6- or 7-SNP GRS to either an internally derived routine basis model or the aMAP score.

Table 6. Features associated with HCC occurrence in univariate analysis. AFP, alpha-fetoprotein; ALT, alanine aminotransferase; AST, aspartate aminotransferase; BMI, body mass index; GGT, γ-glutamyltransferase; PT, prothrombin time

| | No HCC N=1059 | HCC N=86 | SHR [95% CI] | Pvalue |
|---|---|---|---|---|
| Age (years) | 58 [51-64] | 61 [55 - 70] | 1.04 [1.02 ; | 0.001 |
| | **No HCC N=1059** | **HCC N=86** | **SHR [95% CI]** | **Pvalue** |
| | | | 1.07] | |
| Male gender | 684 (64.6) | 70 (81.4) | 2.38 [1.39; 4.09] | 0.002 |
| BMI ($kg/m^2$) | 26.3 [23.5 - 30.1] | 27.8 [24.6 - 31.9] | 1.05 [1.01 ; 1.090] | 0.025 |
| BMI ($kg/m^2$) | | | | 0.173 |
| <25 | 313 (37.7) | 20 (26.7) | Ref | |
| [25 ; 30[ | 304 (36.6) | 29 (38.7) | 1.37 [0.78 ; 2.41] | 0.276 |
| ≥30 | 214 (25.7) | 26 (34.6) | 1.75 [0.97 ; 3.14] | 0.061 |
| Diabetes | 208 (19.6) | 32 (37.7) | 2.29 [1.48 ; 3.54] | <0.001 |
| Past excessive alcohol consumption | 614 (60.0) | 63 (74.1) | 1.57 [0.97 ; 2.55] | 0.068 |
| Platelet counts ($10^3/mm^3$) | 149 [103 - 197] | 105.5 [79.5 - 151.5] | 0.991 [0.986 ; 0.995] | <0.001 |

(continued)

| | No HCC N=1059 | HCC N=86 | SHR [95% CI] | Pvalue |
|---|---|---|---|---|
| AST (IU/L) | 32 [25 - 46] | 36 [29 - 53] | 1.004 [1.001 ; 1.008] | **0.026** |
| AST $\times$ N (N=40) | 0.80 [0.63 - 1.15] | 0.90 [0.73 - 1.33] | 1.19 [1.02; 1.39] | **0.026** |
| ALT (IU/L) | 27 [20 - 41.5] | 29 [22 - 42] | 1.004 [0.999; 1.009] | 0.098 |
| ALT $\times$ N (N=40) | 0.68 [0.50 - 1.04] | 0.73 [0.55 - 1.05] | 1.17 [0.97; 1.41] | 0.098 |
| GGT (IU/L) | 63.0 [36.0 - 140.0] | 131.5 [64.0 - 256.0] | 1.000 [0.999 ; 1.000] | 0.083 |
| GGT $\times$ N (N=45) | 1.40 [0.80 - 3.11] | 2.92 [1.42 - 5.69] | 1.01 [0.99 ; 1.01] | 0.083 |
| PT (%) | 85 [73 - 96] | 76 [70 - 84] | 0.98 [0.97 ;0.99] | **<0.001** |
| Serum albumin | 41.8 [38.0 - | 39.7 [36.4 - | 0.92 [0.88 ; | **<0.001** |
| (g/L) | 44.5] | 42.1] | 0.95] | |
| Total bilirubin ($\mu$mol/L) | 11.0 [8.0 - 17.0] | 15.0 [9.1 - 20.0] | 1.03 [1.01 ; 1.05] | **<0.001** |
| AFP (ng/mL) | 3.7 [2.4 - 5.5] | 4.0 [2.8 - 6.3] | 1.000 [0.999; 1.002] | 0.626 |
| aMAP score | 58.1 [52.8 - 62.9] | 64.0 [60.6 - 68.1] | 1.14 [1.09; 1.19] | **<0.001** |
| Cirrhosis aetiology | | | | |
|     HCV cured | 622 (58.7) | 37 (43.0) | Ref | |
|     Alcohol | 437 (41.3) | 49 (57.0) | 1.50 [0.98 ; 2.29] | 0.062 |
| **6 SNPs-GRS** | | | | **0.014** |
|     0-4 | 344 (32.5) | 19 (22.1) | Ref | |
|     5-6 | 574 (54.2) | 48 (55.8) | 1.54 [0.91 ; 2.62] | 0.110 |
|     7-11 | 141 (13.3) | 19 (22.1) | 2.58 [1.36 ; 4.88] | **0.004** |
| **7 SNPs-GRS** | | | | **0.001** |
|     0-6 | 594 (56.1) | 33 (38.4) | Ref | |
|     7 | 252 (23.8) | 24 (27.9) | 1.73 [1.03; 2.92] | **0.040** |
|     8-13 | 213 (15.3) | 29 (33.7) | 2.50 [1.52 ; 4.11] | **0.004** |

Using Fine-Gray multivariate analyses, both 6- and 7-SNP GRS were independently associated with a higher HCC risk regardless of the applied clinical scoring system. When applied to the CIRRAL and CirVir cohorts, the 7-SNP GRS was the only selected GRS selected by the multivariate models to be associated with HCC, an effect which was only restricted to the CIRRAL cohort regardless of routine or aMAP score.

Table 7 shows the clinical characteristics of patients as a function of the 7-SNP GRS. Overall, patients with the highest scores were older, had higher liver test alterations, and more pronounced signs of severe liver disease. These patients also had the highest aMAP scores.

Table 7. Characteristics of patients as a function of the 7 SNPs-genetic risk score. AFP, alpha-fetoprotein; ALT, alanine aminotransferase; AST, aspartate aminotransferase; BMI, body mass index; GGT, γ-glutamyltransferase; PT, prothrombin time

| | Availa ble data | Scores 0-6 N=627 | Score 7 N=276 | Scores 8-13 N=242 | Pvalue |
|---|---|---|---|---|---|
| Age (years) | 1145 | 58 [52-65] | 57 [50-63.5] | 59 [52-66] | **0.032** |
| Male gender | 1145 | 405 (64.6) | 181 (65.6) | 168 (69.4) | 0.402 |
| BMI (kg/m$^2$) | 906 | 27.0 [23.2 - 30.5] | 26.1 [23.7 - 29.7] | 26.3 [23.7 - 30.0] | 0.475 |
| Platelet counts ($10^3$/mm$^3$) | 1056 | 148 [103 - 202] | 146 [98 - 192] | 144.5 [97 - 182] | 0.196 |
| AST (IU/L) | 1054 | 31 [25 - 44] | 33 [26 - 48] | 35 [27 - 49] | **0.007** |
| AST × $N$ ($N$=40) | 1054 | 0.78 [0.63 - 1.10] | 0.83 [0.65 - 1.20] | 0.88 [0.68 - 1.23] | **0.007** |
| ALT (IU/L) | 1058 | 26 [19 - 40] | 29 [22 - 42] | 32 [23 - 46] | **<0.001** |
| ALT × $N$ ($N$=40) | 1058 | 0.65 [0.48 - 1.00] | 0.73 [0.55 - 1.05] | 0.80 [0.58 - 1.15] | **<0.001** |
| GGT (IU/L) | 998 | 61 [35 - 145] | 78 [41 - 151] | 72.5 [41 - 154] | 0.110 |
| GGT x $N$ ($N$=45) | 998 | 1.36 [0.78 - 3.22] | 1.73 [0.91 - 3.36] | 1.61 [0.91 - 3.42] | 0.110 |
| PT (%) | 939 | 86 [75 - 97] | 81.5 [70 - 94] | 81.5 [72 - 94] | **0.003** |
| Serum albumin (g/L) | 902 | 41.7 [38.2 - 44.0] | 41.4 [37.9 - 44.6] | 41.4 [37.4 - 44.5] | 0.677 |
| Total bilirubin | 975 | 11.0 [8.0 - | 12.0 [8.0 - | 12.0 [8.0 - | 0.174 |
| | Availa ble data | Scores 0-6 N=627 | Score 7 N=276 | Scores 8-13 N=242 | Pvalue |
| (μmol/L) | | 16.9] | 19.0] | 18.3] | |
| AFP (ng/mL) | 824 | 3.5 [2.2 - 5.6] | 3.8 [2.4 - 5.4] | 4.0 [2.9 - 6.0] | 0.159 |
| aMAP score | 840 | 58.4 [52.7 - 63.2] | 58.2 [53.3 - 62.8] | 60.0 [55.1 - 64.8] | **0.015** |
| Cirrhosis aetiology | 1145 | | | | **0.035** |
| HCV cured Alcohol | | 379 (60.5) 248 (39.5) | 157 (56.9) 119 (43.1) | 123 (50.8) 119 (49.2) | |

<u>HCC risk stratification model performance and decision curve analyses</u>

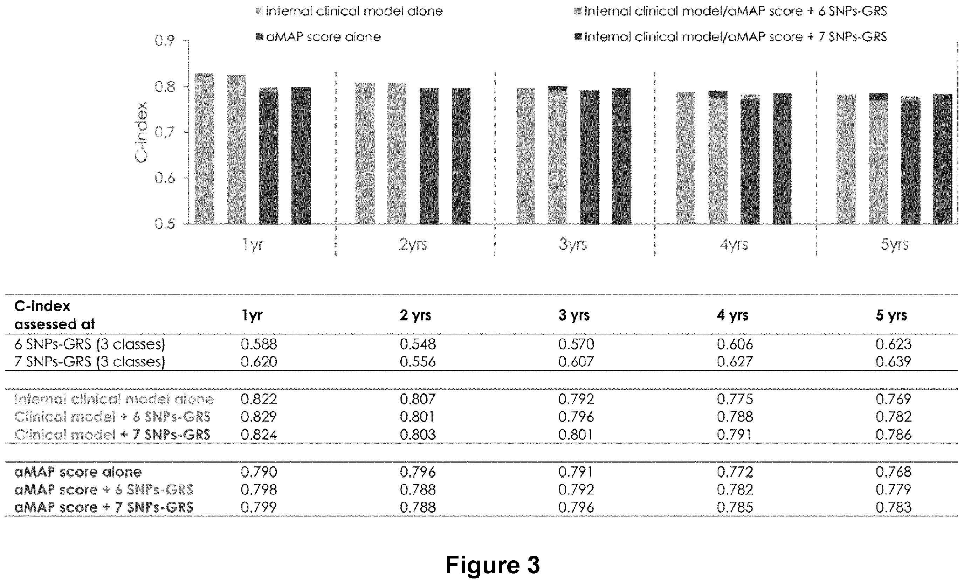

Figure 3 shows the discriminative performances of internal and aMAP scores alone or following the incorporation of the 6-SNP or 7-SNP GRS. The internally derived routine model yielded a C-index of 0.769 for 5-year HCC risk prediction. When incorporating the genetic features into the model, the C-index increased to 0.782 after adding the 6-SNP GRS and to 0.786 after adding the 7-SNP GRS.

Similarly, the aMAP model yielded a C-index of 0.768 for 5-year HCC risk prediction. When incorporating the genetic features into the model, the C-index increased to 0.779 after adding the 6-SNP GRS and to 0.783 after adding the 7-SNP GRS. Similar trends were observed when restricting the analyses to the CIRRAL and CirVir cohorts (see Tables 8 and 9).

Table 9. Performances of HCC prediction models in the CirVir cohort

|  | Internal clinical model alone | Clinical model + 6 SNPs-GRS | Clinical model + 7 SNPs-GRS |
|---|---|---|---|
| C-index 1 yr | 0.747 | 0.732 | 0.737 |
| C-index 2 yrs | 0.830 | 0.823 | 0.807 |
| C-index 3 yrs | 0.834 | 0.834 | 0.833 |
| C-index 4 yrs | 0.852 | 0.855 | 0.854 |
| C-index 5 yrs | 0.810 | 0.814 | 0.824 |

Table 8. Performances of HCC prediction models in the CIRRAL cohort

|  | aMAP score alone | aMAP score + 6 SNPs-GRS | aMAP score + 7 SNPs-GRS |
|---|---|---|---|
| C-index 1 yr | 0.786 | 0.765 | 0.773 |
| C-index 2 yrs | 0.811 | 0.801 | 0.788 |
| C-index 3 yrs | 0.829 | 0.831 | 0.827 |
| C-index 4 yrs | 0.840 | 0.846 | 0.841 |
| C-index 5 yrs | 0.799 | 0.808 | 0.815 |

Figure 7 shows the comparison of cumulative incidence of 5-yrs HCC for high/low-risk patients, defined by clinical model vs clinical model+7-SNPs GRS. High/low-risk was defined according to illustrative percentile cut-off points. Figure 8 shows similar approach using the aMAP score. Overall, the addition of the 7-SNPs GRS to the internal clinical model or the aMAP score slightly improved the discrimination of high/low-risk patients.

Decision curves were finally plotted to test the clinical utility of the 7-SNP GRS alone or as a refinement of the internal routine model or the aMAP score for 3- or 5-year HCC risk prediction. The 7-SNP GRS alone showed the weakest net benefit compared with the two routine models. When the latter were applied, their clinical utility was only modestly improved by the adjunction of the 7-SNP GRS.

Similar trends were observed when restricting the analyses to the CIRRAL and CirVir cohorts.

## Example 3. Discussion

The results reported above, based on the analysis of large prospective cohorts of patients included in HCC surveillance programs with extensive bioclinical characterization, long follow-up and prospective analysis of events based on patients medical files allows us to draw several conclusions. First, a GRS impacting lipid metabolism exerts an independent predictive value on HCC development. Second, the adjunction of the recently identified locus, involved in the Wnt-β-catenin pathway, increased the performance of this GRS. Third, the incorporation of this genetic information into clinical models modestly improves HCC risk stratification.

Hepatic fat content has been shown to be influenced by genetic variants [11], the latter being associated with the presence of HCC in European populations [8-10]. However, these case-control approaches included heterogeneous populations comprising healthy individuals or patients with mild forms of liver diseases who are not the target for HCC surveillance, thus introducing several interpretation biases. In contrast, the assessment of these SNPs in the present longitudinal cohorts provides robust arguments suggesting a direct link with liver carcinogenesis. Indeed, the study of patients with alcoholic and cured HCV showed that PNPLA3 and MBOAT7 (to a lesser extent) exerted the highest oncogenic effect when both cohorts were combined (Table 2), reflecting the selective influence of lipid metabolism on the liver oncogenic process in patients in whom the pro-carcinogenic effect of viral replication has been suppressed; indeed, the association of liver-fat modulating SNPs with HCV-related HCC is debated (ref). However, this inconclusive observation holds true in patients with active HCV replication: recent longitudinal studies conducted in patients who achieved SVR have indeed suggest that this genetic background may impact HCC occurrence. This observation reinforces the hypothesis to consider patients with alcoholic and/or metabolic and/or cured HCV-related cirrhosis as a "universal" phenotype, particularly given the high prevalence of excessive alcohol consumption (more than 30%) or features of metabolic syndrome (nearly 60%) in HCV-cured patients (see Table 1). Consequently, based on the rigorous clinical definition of included patients (biopsy-proven cirrhosis, exclusion of patients with active viral replication, extensive clinical description, protocolized monitoring), the 6-SNP GRS fairly stratified this population into different HCC risk classes (Figure 2A). Moreover, all outcomes were considered during a long follow-up allowing to consider HCC development in a competing risks framework; in this context, the 6-SNP GRS did not impact non-HCC mortality despite an association with a more pronounced liver function impairment (see Suppl Table 3 and Suppl Figure 4A). Finally, this GRS was an independent factor associated with HCC occurrence (Table 4). Taken together, these observations provide the strongest clinical arguments to date for the direct impact of this genetic heterogeneity on liver cancer development.

Following the recent identification of a new HCC susceptibility locus affecting the Wnt-β-catenin pathway [12], we sought to investigate its additional impact on HCC occurrence. Indeed, this GWAs highlighted an additional genetic variation in the WNT3A-WNT9A locus modifying the HCC risk in patients with ALD. The rs708113[T] allele was associated with lower rates of HCC in these patients, an effect that seemed independent from liver fibrosis status and suggested a more direct effect on liver carcinogenesis compared with SNPs modulating hepatic fat content. Translational experiments suggested the promotion of a liver inflammatory environment by the rs708113[T] allele, which may prevent the activation of oncogenic β-catenin, thus decreasing the cancerization process. The present report confirms this specific association by externally validating the impact of WNT3A-WNT9A rs708113 on HCC occurrence in the CIRRAL cohort. When considering the whole population, the addition of WNT3A-WNT9A rs708113 to the four aforementioned variants improved HCC risk stratification through higher SHRs (Figure 2B).

The extent to which GRS may impact clinical practice deserves to be proven. For that matter, one must not only consider genetic factors but also simple routine parameters already known to accurately stratify patients who are eligible for HCC surveillance into various risk classes. Several clinical scoring systems have been developed [35], and the widespread use of antivirals has led to the construction of simple scores that can be applied to patients without viral replication regardless of the cause of liver disease [18]. In this context, we constructed an internal model using results of the multivariate model and also applied the previously developed aMAP score as an external model.[19] Both models performed well in this population, with a similar 5-yr C-Index of 0.769. When enriched by 6- or 7-SNP GRS, both models performed better, but this improvement was modest. This observation was further strengthened by decision-curve analyses, which confirmed the modest improvement of both internal and external clinical scoring systems incorporating the 7-SNP GRS (Figure 4). Our results are in line with a recent report conducted in patients with HCV-cured cirrhosis, albeit HCC occurrence was not the specific studied outcome (ref). A similar analysis was performed in nearly 200,000 UK biobank participants, which evaluated the enrichment of several liver prognostic scoring systems by several SNPs [36]; although the outcome was also a mixed endpoint encompassing "liver-related complications", this large-scale study showed that the most performant scores were similarly only marginally improved by the adjunction of genetic variants. Nevertheless, other analyses conducted in the very same UK biobank yielded opposite conclusions: this fact once again highlights the pivotal role of prospective cohorts of patients with pre-defined outcomes and events accurately recorded in clinical centres for delineating the basis of future precision medicine.

The main limitation of our study is underlined by potential underpowered analyses when stratified by liver disease as suggested by the mild predictive value of PNPLA3 and MBOAT7 genotypes in the CIRRAL and CirVir cohorts independently and a stronger one when both populations were combined (see Table 2). The same observation was made for the different GRS: when the cohorts were considered separately, the revised 7-SNPs GRS was the only informative genetic score, an effect which was restricted to the CIRRAL cohort (see Figures 2 and 3). After association of the two cohorts, the revised 6-SNPs combining only liver-fat modulating SNPs was clearly associated with HCC occurrence. Similar observations were made when multivariate analyses were performed. Indeed, the revised 6-SNPs combining only liver-fat modulating SNPs was (not surprisingly) not associated with HCC in neither the CirVir or CIRRAL cohorts while it was highlighted as an independent risk factor whether considering the internal or external aMAP clinical scoring system. This fact is partially the consequence of the cautious selection of patients from both cohorts (see Figure 1). In addition, while the prospective design of these longitudinal cohorts limits the ability to follow-up large numbers of patients using standardized surveillance protocols recorded in clinical centers in the long term, such rigorous approach provides the strongest confidence in the drawn conclusions available in this field of research. This clinical approach is in sharp contrast with the aforementioned registry studies [8, 36], which in turn suffer from limited clinical information and outcomes. These statistical issues provide further justification to combine patients with cirrhosis and HCV eradication with other causes on non-viral liver diseases, as highlighted by the development of universal scoring systems such as the aMAP score (ref). While limited longitudinal single-centre studies comprising adjoing biobanks are emerging [37], prospective and protocolized multicentric efforts similar to the CirVir and CIRRAL cohorts are currently ongoing in other countries and will ultimately allow the refinement of our observations when they will be made available. In this setting, the extent to which our findings obtained in a population comprising a large majority of patients of European ancestry could be replicated in other parts of the world deserves to be tested. Ultimately, ongoing international efforts gathering large-scale longitudinal cohorts of patients recruited in European countries will provide further insight on both HCC genetic predisposition and risk stratification.

In summary, patients with cirrhosis included in HCC surveillance programs can be stratified by genetic scores using variants affecting lipid turnover and the Wnt-β-catenin pathway into various HCC risk classes. This genetic information modestly improves the performance of clinical scores for HCC risk allocation. The continuous enrichment with yet to be identified or validated circulating biological components (genetic or not) might ultimately pave the way for personalization of HCC surveillance using more effective tools in a cost-effective fashion, if they are proven to substantially improve the performance of routine scoring systems. In the meantime, ongoing randomized clinical trials (RCTs) aimed at gathering clinical evidence for the benefits of HCC risk-based screening interventions solely rely on clinical scoring systems.

## Conclusion

**Background and aims:** The results reported herein aimed at evaluating the ability of combinations of single nucleotide polymorphisms (SNPs) to refine hepatocellular carcinoma (HCC) risk stratification. **Methods:** Six SNPs in *PNPLA3, TM6SF2, HSD17B13, APOE,* and *MBOAT7* affecting lipid turnover and one variant involved in the Wnt-β-catenin pathway (*WNT3A-WNT9A* rs708113) were assessed in patients with alcohol-related and/or HCV-cured cirrhosis included in HCC surveillance programs (prospective CirVir and CIRRAL cohorts). Their prognostic value for HCC occurrence was assessed using Fine-Gray models combined into a 7-SNP genetic risk score (GRS). Prediction ability of two clinical scores (a routine nongenetic model determined by multivariate analysis and the external aMAP score) without then with the addition of the GRS was evaluated by C-indices. The standardized net benefit was derived from decision curves. **Results:** Among 1145 patients, 86 (7.5%) developed HCC after 66.3 months. *PNPLA3* and *WNT3A-WNT9A* variants were independently associated with HCC occurrence. The GRS stratified the population into 3 groups with progressively increased 5-yr HCC incidence [Group 1 (n=627, 5.4%), Group 2 (n=276, 10.7%), and Group 3 (n=242, 15.3%); P<0.001]. The multivariate model identified age, male sex, diabetes, platelet count, GGT levels, albuminemia and the GRS as independent risk factors. The clinical model performance for 5-yr HCC prediction was similar to that of the aMAP score (C-Index 0.769). The addition of the GRS to both scores modestly improved their performance (C-Index 0.786 and 0.783, respectively). This finding was confirmed by decision curve analyses showing only fair clinical net benefit. **Interpretation:** Patients with cirrhosis can be stratified into HCC risk classes by variants affecting lipid turnover and Wnt-β-catenin pathway. The incorporation of this genetic information improves the performance of clinical scores.

## REFERENCES

[1] Nahon P, Zucman-Rossi J. Single nucleotide polymorphisms and risk of hepatocellular carcinoma in cirrhosis. J Hepatol 2012;57:663-674.

[2] Romeo S, Kozlitina J, Xing C, Pertsemlidis A, Cox D, Pennacchio LA, et al. Genetic variation in PNPLA3 confers susceptibility to nonalcoholic fatty liver disease. Nat Genet 2008;40:1461-1465.

[3] Kozlitina J, Smagris E, Stender S, Nordestgaard BG, Zhou HH, Tybjaerg-Hansen A, et al. Exome-wide association study identifies a TM6SF2 variant that confers susceptibility to nonalcoholic fatty liver disease. Nat Genet 2014;46:352-356.

[4] Abul-Husn NS, Cheng X, Li AH, Xin Y, Schurmann C, Stevis P, et al. A Protein-Truncating HSD17B13 Variant and Protection from Chronic Liver Disease. The New England journal of medicine 2018;378:1096-1106.

[5] Buch S, Stickel F, Trepo E, Way M, Herrmann A, Nischalke HD, et al. A genome-wide association study confirms PNPLA3 and identifies TM6SF2 and MBOAT7 as risk loci for alcohol-related cirrhosis. Nat Genet 2015;47:1443-1448.

[6] Nahon P, Allaire M, Nault JC, Paradis V. Characterizing the mechanism behind the progression of NAFLD to hepatocellular carcinoma. Hepat Oncol 2020;7:HEP36.

[7] Nahon P, Nault JC. Constitutional and functional genetics of human alcohol-related hepatocellular carcinoma. Liver international : official journal of the International Association for the Study of the Liver 2017;37:1591-1601.

[8] Gellert-Kristensen H, Richardson TG, Davey Smith G, Nordestgaard BG, Tybjaerg-Hansen A, Stender S. Combined Effect of PNPLA3, TM6SF2, and HSD17B13 Variants on Risk of Cirrhosis and Hepatocellular Carcinoma in the General Population. Hepatology 2020;72:845-856.

[9] Bianco C, Jamialahmadi O, Pelusi S, Baselli G, Dongiovanni P, Zanoni I, et al. Non-invasive stratification of hepatocellular carcinoma risk in non-alcoholic fatty liver using polygenic risk scores. J Hepatol 2021;74:775-782.

[10] Yang J, Trepo E, Nahon P, Cao Q, Moreno C, Letouze E, et al. A 17-Beta-Hydroxysteroid Dehydrogenase 13 Variant Protects From Hepatocellular Carcinoma Development in Alcoholic Liver Disease. Hepatology 2019;70:231-240.

[11] Trepo E, Valenti L. Update on NAFLD genetics: From new variants to the clinic. J Hepatol 2020;72:1196-1209.

[12] Trepo E, Caruso S, Yang J, Imbeaud S, Couchy G, Bayard Q, et al. Common genetic variation in alcohol-related hepatocellular carcinoma: a case-control genome-wide association study. The Lancet Oncology 2022;23:161-171.

[13] Singal AG, Lampertico P, Nahon P. Epidemiology and surveillance for hepatocellular carcinoma: New trends. J Hepatol 2020;72:250-261.

[14] Singal AG, Zhang E, Narasimman M, Rich NE, Waljee AK, Hoshida Y, et al. HCC Surveillance Improves Early Detection, Curative Treatment Receipt, and Survival in Patients with Cirrhosis: A Systematic Review and Meta-Analysis. J Hepatol 2022;2022 Feb 6:S0168-8278(22)00068-X.

[15] Audureau E, Carrat F, Layese R, Cagnot C, Asselah T, Guyader D, et al. Personalized surveillance for hepatocellular carcinoma in cirrhosis - using machine learning adapted to HCV status. J Hepatol 2020;73:1434-1445.

[16] Kim SY, An J, Lim YS, Han S, Lee JY, Byun JH, et al. MRI With Liver-Specific Contrast for Surveillance of Patients With Cirrhosis at High Risk of Hepatocellular Carcinoma. JAMA oncology 2017;3:456-463.

[17] Nahon P, Najean M, Layese R, Zarca K, Segar LB, Cagnot C, et al. Early hepatocellular carcinoma detection using magnetic resonance imaging is cost-effective in high-risk patients with cirrhosis. JHEP Rep 2022;4:100390.

[18] Nahon P, Vo Quang E, Ganne-Carrie N. Stratification of Hepatocellular Carcinoma Risk Following HCV Eradication or HBV Control. J Clin Med 2021;10(2):353..

[19] Fan R, Papatheodoridis G, Sun J, Innes H, Toyoda H, Xie Q, et al. aMAP risk score predicts hepatocellular carcinoma development in patients with chronic hepatitis. J Hepatol 2020;73:1368-1378.

[20] Trinchet JC, Bourcier V, Chaffaut C, Ait Ahmed M, Allam S, Marcellin P, et al. Complications and competing risks of death in compensated viral cirrhosis (ANRS CO12 CirVir prospective cohort). Hepatology 2015;62:737-750.

[21] Ganne-Carrie N, Chaffaut C, Bourcier V, Archambeaud I, Perarnau JM, Oberti F, et al. Estimate of hepatocellular carcinoma incidence in patients with alcoholic cirrhosis. J Hepatol 2018;69:1274-1283.

[22] Bruix J, Sherman M. Management of hepatocellular carcinoma. Hepatology 2005;42: 1208-1236.

[23] Bruix J, Sherman M. Management of hepatocellular carcinoma: an update. Hepatology 2011;53:1020-1022.

[24] EASL Clinical Practice Guidelines: Management of hepatocellular carcinoma. J Hepatol 2018;69:182-236.

[25] Nahon P, Lescat M, Layese R, Bourcier V, Talmat N, Allam S, et al. Bacterial infection in compensated viral cirrhosis impairs 5-year survival (ANRS CO12 CirVir prospective cohort). Gut 2017;66:330-341.

[26] Costentin CE, Layese R, Bourcier V, Cagnot C, Marcellin P, Guyader D, et al. Compliance With Hepatocellular Carcinoma Surveillance Guidelines Associated With Increased Lead-Time Adjusted Survival of Patients With Compensated Viral Cirrhosis: A Multi-Center Cohort Study. Gastroenterology 2018;155:431-442 e410.

[27] Allaire M, Nahon P, Layese R, Bourcier V, Cagnot C, Marcellin P, et al. Extrahepatic cancers are the leading cause of death in patients achieving hepatitis B virus control or hepatitis C virus eradication. Hepatology 2018;68:1245-1259.

[28] Cacoub P, Nahon P, Layese R, Blaise L, Desbois AC, Bourcier V, et al. Prognostic value of viral eradication for major adverse cardiovascular events in hepatitis C cirrhotic patients. American heart journal 2018;198:4-17.

[29] Ganne-Carrie N, Nahon P, Chaffaut C, N'Kontchou G, Layese R, Audureau E, et al. Impact of cirrhosis aetiology on incidence and prognosis of hepatocellular carcinoma diagnosed during surveillance. JHEP Rep 2021;3:100285.

[30] Nahon P, Bourcier V, Layese R, Audureau E, Cagnot C, Marcellin P, et al. Eradication of Hepatitis C Virus Infection in Patients With Cirrhosis Reduces Risk of Liver and Non-Liver Complications. Gastroenterology 2017;152:142-156 e142.

[31] Park SH, Kim S. Pattern discovery of multivariate phenotypes by association rule mining and its scheme for genome-wide association studies. Int J Data Min Bioinform 2012;6:505-520.

[32] Wolbers M, Blanche P, Koller MT, Witteman JC, Gerds TA. Concordance for prognostic models with competing risks. Biostatistics 2014;15:526-539.

[33] Vickers AJ, Elkin EB. Decision curve analysis: a novel method for evaluating prediction models. Med Decis Making 2006;26:565-574.

[34] Vickers AJ, Van Calster B, Steyerberg EW. Net benefit approaches to the evaluation of prediction models, molecular markers, and diagnostic tests. BMJ 2016;352:i6.

[35] Sherman M. HCC Risk Scores: Useful or Not? Seminars in liver disease 2017;37:287 -295.

[36] Innes H, Morling JR, Buch S, Hamill V, Stickel F, Guha IN. Performance of routine risk scores for predicting cirrhosis-related morbidity in the community. J Hepatol 2022;Mar 7;S0168-8278(22)00129-5..

[37] Degasperi E, Galmozzi E, Pelusi S, D'Ambrosio R, Soffredini R, Borghi M, et al. Hepatic Fat-Genetic Risk Score Predicts Hepatocellular Carcinoma in Patients With Cirrhotic HCV Treated With DAAs. Hepatology 2020;72:1912-1923.

[38] Innes H, Nischalke HD, Guha IN, Weiss KH, Irving W, Gotthardt D, et al. The rs429358 Locus in Apolipoprotein E Is Associated With Hepatocellular Carcinoma in Patients With Cirrhosis. Hepatol Commun 2022;6:1213-1226.

[39] Jamialahmadi O, Mancina RM, Ciociola E, Tavaglione F, Luukkonen PK, Baselli G, et al. Exome-Wide Association Study on Alanine Aminotransferase Identifies Sequence Variants in the GPAM and APOE Associated With Fatty Liver Disease. Gastroenterology 2021;160:1634-1646 e1637.

**Claims**

1. An ex *vivo* method for determining whether a patient with cirrhosis or advanced chronic liver disease has an increased risk of developing hepatocarcinoma cancer (HCC) within a given period of time, comprising:

    a) providing the values of the concentration of at least two biochemical markers in the blood, serum or plasma of the patient

    b) determining the presence or absence of alleles specifically associated with HCC for at least two genes associated with HCC in the genome of the patient, allocating a numerical value depending on the presence of none, one or two alleles specifically associated with HCC in the genome of the patient, and combining the numerical values through a mathematical function for each gene to obtain an intermediate value

    c) combining the values of a) and the numerical values or the intermediate value of b) in a mathematical function, so as to obtain an end value

    d) comparing the end value to a predetermined value,

    wherein the patient has an increased risk of developing HCC if the end value is higher than the predetermined value.

2. The method of claim 1, wherein the sex (scored as 0 for female and 1 for male), diabetes status (0 for absence, 1 for presence) and the age of the patient (in years) are also combined in the function of c).

3. The method of claim 1 or 2, wherein the biochemical markers are selected from α2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, γ-globulin, albumin, alpha fetoprotein (AFP), α1-globulin, α2-globulin, β-giobuhn, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructosebisphosphate aldolase B, fumarylacetoacetase, transthyretin, PR02275, Creactive protein (isoform 1), leucine-rich alpha-2-glycoprotein, serpin A11, DNA-directed RNA polymerase I subunit RPA1, obscurin (isoform 1), alphaskeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1, carbohydrate deficient transferrin, α-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), α-Lfucosidase (AFU), Des-γ-carboxyprothrombin (DCP or PIVKA), Osteopontin (OPN), and Human Carbonyl Reductase.

4. The method of any one of claims 1 to 3, wherein the biochemical markers are platelet count, GGT levels, albuminemia.

5. The method of any one of claims 1 to 4, wherein the genes associated with HCC are selected from the group consisting of genes coding for *PNPLA3, TM6SF2, HSD17B13, MBOAT7* and *WNT3A-WNT9A* (rs708113) CYP2R1, DDX18 region, DEPDC5, DHCR7, DLC1, EGF, ESR1 Pvull CG, GFRA1, GRIK1, HCP5 (MICA region), HFE C282Y, IL-28B C/T, IL1α Indel (miR-122), IL1β C-31T , IL1β C-511T , IL6 C-174G, MDM2 G-309T, MICA region, miR-146a GC, MTHFR A-1298C, MTHFR C-677T, NFKβIA G-881A, P53 Arg72 Pro, RANTES G-403A, SCYB14, SOD2 A16V, STAT3 intron 11 G/C, STAT4, TEP1, TERF1, TERT, TGFβ 1, TLR4 G/T, TNF G-308A, TNFα G-238A, UBE4B-KIF1B-PGD region, UGT1A7 N129K W208R, UGT1A7 R131K, UGT1A7 R131K N129K, XPC L-939G, XRCC3 C18067T and XRCC4.

6. The method of any one of claims 1 to 5, wherein, for each gene associated with HCC, the value 0 is allocated when the patient has no allele specifically associated with HCC, the value 1 is allocated when the patient has one allele specifically associated with HCC and one allele that is not specifically associated with HCC, and the value 2 is allocated when the patient is homozygous for an allele specifically associated with HCC or contains two alleles specifically associated with HCC.

7. The method of any one of claims 1 to 6, wherein the function has been obtained by

a) providing the concentration of biochemical markers as measured in the blood, serum or plasma of patients of a cohort of patients;

b) determining the presence or absence of alleles specifically associated with HCC in the genome of the patient in genes associated with HCC, wherein the alleles of at least two genes are determined, allocating a numerical value depending on the presence of none, one or two alleles specifically associated with HCC in the genome of the patient, and combining the numerical values through a mathematical function for each gene to obtain an intermediate value;

c) performing a follow-up of the patients of the cohort during the given period of time;

d) determining the occurrence of HCC for each patient of the cohort during the given period of time;

e) classifying the patients of the cohort in different groups according to the occurrence of HCC during the given period of time;

f) identifying the biochemical markers which differ significantly between these groups by unidimensional analysis;

g) performing a regression analysis to assess the independent discriminative value of the markers for the occurrence of HCC cancer during the given period of time;

h) obtaining the function by combination of the independent markers identified in g), of the numerical value obtained in b), and optionally of the age, diabetes status, sex and/or Body Mass Index of the patient.

8. The method of any one of claims 1 to 7, wherein the function has been obtained by Fine-Gray regression modelling.

9. The method of any one of claims 1 to 9, wherein the given period of time is five years.

10. The method of any one of claims 1 to 9, wherein the given period of time is one year.

11. The method of any one of claims 1 to 10, wherein the function is

$$a1 \times \text{age (years)} + a2 \times \text{Sex} + a3 \times \text{Diabetes} - a4 \times \text{platelet counts} (10^3/\text{mm}^3) + a5 \times \text{GGT} (xN) - a6 \times \text{serum albumin} (g/L) + a7 \times 7\text{SNPsGRS}_7 + a8 \times 7\text{SNPsGRS}_{8\text{-}13}$$

with

- $0.038 \leq a1 \leq 0.045$, preferably $0.040 \leq a1 \leq 0.042$
- $0.92 \leq a2 \leq 1.02$, preferably $0.94 \leq a2 \leq 1.00$
- $0.45 \leq a3 \leq 0.55$, preferably $0.48 \leq a3 \leq 0.52$
- $0.007 \leq a4 \leq 0.009$, preferably $0.075 \leq a4 \leq 0.080$
- $0.005 \leq a5 \leq 0.015$, preferably $0.008 \leq a5 \leq 0.013$
- $0.040 \leq a6 \leq 0.053$, preferably $0.043 \leq a6 \leq 0.050$
- $0.40 \leq a7 \leq 0.60$, preferably $0.45 \leq a7 \leq 0.55$
- $0.68 \leq a8 \leq 0.82$, preferably $0.72 \leq a8 \leq 0.78$,

wherein Male gender, Diabetes equal to 1 when present, and to 0 otherwise (for female of no diabetes); $7\text{SNPsGRS}_7$ ($7\text{SNPsGRS}_{class1}$) equals to 1 when the sum of 7 alleles scores equals to 7, and to 0 otherwise; and $7\text{SNPsGRS}_{8\text{-}13}$ ($7\text{SNPsGRS}_{class2}$) equals to 1 when the sum of 7 alleles is between 8 and 13, and to 0 otherwise, wherein the GGT is relative to normal (xN = "x times normal") and wherein the SNPs used are rs738409 (PNPLA3), rs58542926 (TM6SF2), rs187429064 (TM6SF2), rs72613567 (HSD17B13), rs429358 (APOE), rs641738 (MBOAT7) and rs708113 (WNT3A-WNT9A locus).

12. The method of any one of claims 1 to 11, wherein the function is

$$Score = 0.04085 * Age_{years} + 0.97209 * Male\ gender + 0.50060$$
$$* Diabetes - 0.0078 * Platelet\ count_{10^3/mm^3}$$
$$+ 0.01167 * GGT_{xN} - 0.04688 * Serum\ albumin_{g/L}$$
$$+ 0.50834 * 7SNPsGRS_7 + 0.75184 * 7SNPsGRS_{8-13}$$

13. A method for determining whether a patient with cirrhosis or advanced chronic liver disease has an increased risk of developing HCC within a given period of time, comprising

   a) Determining the presence or absence of alleles specifically associated with HCC in the genome of the patient in genes associated with HCC, wherein the alleles of at least two genes are determined;
   b) Allocating a numerical value depending on the presence of none, one or two alleles specifically associated with HCC in the genome of the patient;
   c) Combining, in a mathematical function, all the values obtained in b) in order to obtain an end value;
   d) Comparing the end value to a predetermined value;

wherein the patient has an increased risk of developing HCC within the given period of time if the end value is higher than the predetermined value.

14. The method of any one of claims 1 to 13, wherein the combination and the comparison are implemented by a computer.

15. A method for determining whether a patient has an increased risk of developing HCC within a given period of time, comprising

   a) having a sender identify himself to a server within a public or private network,
   b) requiring the sender to fill-in information related to the patient, and assigning a specific identifier to the patient in a database;
   c) obtaining values of the concentration of at least two biochemical markers in the blood, serum or plasma of the patient, and storing the values in the database, associated with the specific identifier of the patient
   d) obtaining information relating to the presence or absence of alleles specifically associated with HCC in the genome of the patient in genes associated with HCC, wherein the alleles of at least two genes are obtained, and storing the information in the database, associated with the specific identifier of the patient
   e) allocating a numerical value depending on the presence of none, one or two alleles specifically associated with HCC in the genome of the patient, and storing the numerical values in the database, associated with the specific identifier of the patient
   f) combining the values of c) and the values of e) in a function to obtain an end value
   g) storing the end value in a database associated with the specific identifier, preferably with the date and time of identification of the sender
   h) comparing the end value to a predetermined value, determining the nature of the risk of the patient (increased risk, lower risk or average risk) of developing an HCC for the patient,
   i) sending the nature of the risk to the sender.

Figure 1

**Figure 2**

| C-index assessed at | 1yr | 2 yrs | 3 yrs | 4 yrs | 5 yrs |
|---|---|---|---|---|---|
| 6 SNPs-GRS (3 classes) | 0.588 | 0.548 | 0.570 | 0.606 | 0.623 |
| 7 SNPs-GRS (3 classes) | 0.620 | 0.556 | 0.607 | 0.627 | 0.639 |
| | | | | | |
| Internal clinical model alone | 0.822 | 0.807 | 0.792 | 0.775 | 0.769 |
| Clinical model + 6 SNPs-GRS | 0.829 | 0.801 | 0.796 | 0.788 | 0.782 |
| Clinical model + 7 SNPs-GRS | 0.824 | 0.803 | 0.801 | 0.791 | 0.786 |
| | | | | | |
| aMAP score alone | 0.790 | 0.796 | 0.791 | 0.772 | 0.768 |
| aMAP score + 6 SNPs-GRS | 0.798 | 0.788 | 0.792 | 0.782 | 0.779 |
| aMAP score + 7 SNPs-GRS | 0.799 | 0.788 | 0.796 | 0.785 | 0.783 |

**Figure 3**

Figure 4

EP 4 353 838 A1

**A**

Cumulative Incidence of HCC

Legend: 0-4, 5-6, ≥7

$P = 0.103$

Time (months): 0 12 24 36 48 60 72 84 96 108 120

**B**

Cumulative Incidence of HCC

Legend: 0-6, 7, ≥8

$P = 0.229$

Time (months): 0 12 24 36 48 60 72 84 96 108 120

**Figure 5**

**Figure 6**

31

**Figure 7**

Figure 7 (continued)

**G.**

**H.**

**I.**

# Figure 7 (continued)

Figure 8

D.

E.

F.

**Figure 8 (continued)**

G.

H.

I.

**Figure 8 (continued)**

Europäisches
Patentamt

European
Patent Office

Office européen
des brevets

# EUROPEAN SEARCH REPORT

**Application Number**

EP 22 30 6522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | INNES HAMISH ET AL: "The rs429358 Locus in Apolipoprotein E Is Associated With Hepatocellular Carcinoma in Patients With Cirrhosis", HEPATOLOGY COMMUNICATIONS, vol. 6, no. 5, 31 May 2021 (2021-05-31), pages 1213-1226, XP093032867, DOI: 10.1002/hep4.1886/suppinfo Retrieved from the Internet: URL:https://www.ncbi.nlm.nih.gov/pmc/articles/PMC9035556/pdf/HEP4-6-1213.pdf> * page 1214 - page 1215 * * page 1224 * * abstract * * page 1217 * | 1-15 | INV. C12Q1/6886 |
| Y,D | AUDUREAU ETIENNE ET AL: "Personalized surveillance for hepatocellular carcinoma in cirrhosis – using machine learning adapted to HCV status", JOURNAL OF HEPATOLOGY, ELSEVIER, AMSTERDAM, NL, vol. 73, no. 6, 29 June 2020 (2020-06-29), pages 1434-1445, XP086344980, ISSN: 0168-8278, DOI: 10.1016/J.JHEP.2020.05.052 [retrieved on 2020-06-29] * figures 1,2; table 3 * | 1-15 | |
| | ----- -/-- | | TECHNICAL FIELDS SEARCHED (IPC) C12Q |

The present search report has been drawn up for all claims

| Place of search | Date of completion of the search | Examiner |
|---|---|---|
| The Hague | 24 March 2023 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIE YANG ET AL: "PNPLA3 and TM6SF2 variants as risk factors of hepatocellular carcinoma across various etiologies and severity of underlying liver diseases", INTERNATIONAL JOURNAL OF CANCER, JOHN WILEY & SONS, INC, US, vol. 144, no. 3, 9 November 2018 (2018-11-09), pages 533-544, XP071290555, ISSN: 0020-7136, DOI: 10.1002/IJC.31910 * pages 534,536; table Supp. 4 * * abstract; figure 1; table 4 * * page 542 * & Yang Jie: "Supplementary table 2", , 9 November 2019 (2019-11-09), XP093034372, Retrieved from the Internet: URL:https://onlinelibrary.wiley.com/action /downloadSupplement?doi=10.1002%2Fijc.3191 0&file=ijc31910-sup-0003-TableS2.docx [retrieved on 2023-03-23] ----- | 1-10, 12-15 | |
| A | WHITFIELD JOHN B ET AL: "A genetic risk score and diabetes predict development of alcohol-related cirrhosis in drinkers", JOURNAL OF HEPATOLOGY, ELSEVIER, AMSTERDAM, NL, vol. 76, no. 2, 14 October 2021 (2021-10-14), pages 275-282, XP086926177, ISSN: 0168-8278, DOI: 10.1016/J.JHEP.2021.10.005 [retrieved on 2021-10-14] * the whole document * ----- | 1-15 | TECHNICAL FIELDS SEARCHED (IPC) |

-/--

The present search report has been drawn up for all claims

| Place of search | Date of completion of the search | Examiner |
|---|---|---|
| The Hague | 24 March 2023 | Reuter, Uwe |

EPO FORM 1503 03.82 (P04C01)

Europäisches
Patentamt

European
Patent Office

Office européen
des brevets

## EUROPEAN SEARCH REPORT

Application Number

EP 22 30 6522

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PATERNOSTRO RAFAEL ET AL: "Combined effects of PNPLA3, TM6SF2 and HSD17B13 variants on severity of biopsy-proven non-alcoholic fatty liver disease", HEPATOLOGY INTERNATIONAL, SPRINGER INDIA, INDIA, vol. 15, no. 4, 2 June 2021 (2021-06-02), pages 922-933, XP037545726, ISSN: 1936-0533, DOI: 10.1007/S12072-021-10200-Y [retrieved on 2021-06-02] * the whole document * | 1-15 | |
| A | BURLONE MICHELA E ET AL: "HSD17B13 and other liver fat-modulating genes predict development of hepatocellular carcinoma among HCV-positive cirrhotics with and without viral clearance after DAA treatment", CLINICAL JOURNAL OF GASTROENTEROLOGY, SPRINGER JAPAN, JAPAN, vol. 15, no. 2, 31 January 2022 (2022-01-31), pages 301-309, XP037751649, ISSN: 1865-7257, DOI: 10.1007/S12328-021-01578-1 [retrieved on 2022-01-31] * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 24 March 2023 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 30 6522

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE VINCENTIS ANTONIO ET AL: "A Polygenic Risk Score to Refine Risk Stratification and Prediction for Severe Liver Disease by Clinical Fibrosis Scores", CLINICAL GASTROENTEROLOGY AND HEPATOLOGY, vol. 20, no. 3, 1 March 2022 (2022-03-01), pages 658-673, XP093034270, AMSTERDAM, NL ISSN: 1542-3565, DOI: 10.1016/j.cgh.2021.05.056 * the whole document * | 1-15 | |

TECHNICAL FIELDS
SEARCHED      (IPC)

The present search report has been drawn up for all claims

| Place of search | Date of completion of the search | Examiner |
|---|---|---|
| The Hague | 24 March 2023 | Reuter, Uwe |

EPO FORM 1503 03.82 (P04C01)

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NAHON P ; ZUCMAN-ROSSI J.** Single nucleotide polymorphisms and risk of hepatocellular carcinoma in cirrhosis. *J Hepatol,* 2012, vol. 57, 663-674
- **ROMEO S ; KOZLITINA J ; XING C ; PERTSEMLIDIS A ; COX D ; PENNACCHIO LA et al.** Genetic variation in PNPLA3 confers susceptibility to nonalcoholic fatty liver disease. *Nat Genet,* 2008, vol. 40, 1461-1465
- **KOZLITINA J ; SMAGRIS E ; STENDER S ; NORDESTGAARD BG ; ZHOU HH ; TYBJAERG-HANSEN A et al.** Exome-wide association study identifies a TM6SF2 variant that confers susceptibility to nonalcoholic fatty liver disease. *Nat Genet,* 2014, vol. 46, 352-356
- **ABUL-HUSN NS ; CHENG X ; LI AH ; XIN Y ; SCHURMANN C ; STEVIS P et al.** A Protein-Truncating HSD17B13 Variant and Protection from Chronic Liver Disease. *The New England journal of medicine,* 2018, vol. 378, 1096-1106
- **BUCH S ; STICKEL F ; TREPO E ; WAY M ; HERRMANN A ; NISCHALKE HD et al.** A genome-wide association study confirms PNPLA3 and identifies TM6SF2 and MBOAT7 as risk loci for alcohol-related cirrhosis. *Nat Genet,* 2015, vol. 47, 1443-1448
- **NAHON P ; ALLAIRE M ; NAULT JC ; PARADIS V.** Characterizing the mechanism behind the progression of NAFLD to hepatocellular carcinoma. *Hepat Oncol,* 2020, vol. 7, HEP36
- **NAHON P ; NAULT JC.** Constitutional and functional genetics of human alcohol-related hepatocellular carcinoma. *Liver international : official journal of the International Association for the Study of the Liver,* 2017, vol. 37, 1591-1601
- **GELLERT-KRISTENSEN H ; RICHARDSON TG ; DAVEY SMITH G ; NORDESTGAARD BG ; TYBJAERG-HANSEN A ; STENDER S.** Combined Effect of PNPLA3, TM6SF2, and HSD17B13 Variants on Risk of Cirrhosis and Hepatocellular Carcinoma in the General Population. *Hepatology,* 2020, vol. 72, 845-856
- **BIANCO C ; JAMIALAHMADI O ; PELUSI S ; BASELLI G ; DONGIOVANNI P ; ZANONI I et al.** Non-invasive stratification of hepatocellular carcinoma risk in non-alcoholic fatty liver using polygenic risk scores. *J Hepatol,* 2021, vol. 74, 775-782
- **YANG J ; TREPO E ; NAHON P ; CAO Q ; MORENO C ; LETOUZE E et al.** A 17-Beta-Hydroxysteroid Dehydrogenase 13 Variant Protects From Hepatocellular Carcinoma Development in Alcoholic Liver Disease. *Hepatology,* 2019, vol. 70, 231-240
- **TREPO E ; VALENTI L.** Update on NAFLD genetics: From new variants to the clinic. *J Hepatol,* 2020, vol. 72, 1196-1209
- **TREPO E ; CARUSO S ; YANG J ; IMBEAUD S ; COUCHY G ; BAYARD Q et al.** Common genetic variation in alcohol-related hepatocellular carcinoma: a case-control genome-wide association study. *The Lancet Oncology,* 2022, vol. 23, 161-171
- **SINGAL AG ; LAMPERTICO P ; NAHON P.** Epidemiology and surveillance for hepatocellular carcinoma: New trends. *J Hepatol,* 2020, vol. 72, 250-261
- **SINGAL AG ; ZHANG E ; NARASIMMAN M ; RICH NE ; WALJEE AK ; HOSHIDA Y et al.** HCC Surveillance Improves Early Detection, Curative Treatment Receipt, and Survival in Patients with Cirrhosis: A Systematic Review and Meta-Analysis. *J Hepatol,* 06 February 2022, (22), 00068-X
- **AUDUREAU E ; CARRAT F ; LAYESE R ; CAGNOT C ; ASSELAH T ; GUYADER D et al.** Personalized surveillance for hepatocellular carcinoma in cirrhosis - using machine learning adapted to HCV status. *J Hepatol,* 2020, vol. 73, 1434-1445
- **KIM SY ; AN J ; LIM YS ; HAN S ; LEE JY ; BYUN JH et al.** MRI With Liver-Specific Contrast for Surveillance of Patients With Cirrhosis at High Risk of Hepatocellular Carcinoma. *JAMA oncology,* 2017, vol. 3, 456-463
- **NAHON P ; NAJEAN M ; LAYESE R ; ZARCA K ; SEGAR LB ; CAGNOT C et al.** Early hepatocellular carcinoma detection using magnetic resonance imaging is cost-effective in high-risk patients with cirrhosis. *JHEP Rep,* 2022, vol. 4, 100390
- **NAHON P ; VO QUANG E ; GANNE-CARRIE N.** Stratification of Hepatocellular Carcinoma Risk Following HCV Eradication or HBV Control. *J Clin Med,* 2021, vol. 10 (2), 353
- **FAN R ; PAPATHEODORIDIS G ; SUN J ; INNES H ; TOYODA H ; XIE Q et al.** aMAP risk score predicts hepatocellular carcinoma development in patients with chronic hepatitis. *J Hepatol,* 2020, vol. 73, 1368-1378

- **TRINCHET JC ; BOURCIER V ; CHAFFAUT C ; AIT AHMED M ; ALLAM S ; MARCELLIN P et al.** Complications and competing risks of death in compensated viral cirrhosis (ANRS CO12 CirVir prospective cohort). *Hepatology,* 2015, vol. 62, 737-750
- **GANNE-CARRIE N ; CHAFFAUT C ; BOURCIER V ; ARCHAMBEAUD I ; PERARNAU JM ; OBERTI F et al.** Estimate of hepatocellular carcinoma incidence in patients with alcoholic cirrhosis. *J Hepatol,* 2018, vol. 69, 1274-1283
- **BRUIX J ; SHERMAN M.** Management of hepatocellular carcinoma. *Hepatology,* 2005, vol. 42, 1208-1236
- **BRUIX J ; SHERMAN M.** Management of hepatocellular carcinoma: an update. *Hepatology,* 2011, vol. 53, 1020-1022
- EASL Clinical Practice Guidelines: Management of hepatocellular carcinoma. *J Hepatol,* 2018, vol. 69, 182-236
- **NAHON P ; LESCAT M ; LAYESE R ; BOURCIER V ; TALMAT N ; ALLAM S et al.** Bacterial infection in compensated viral cirrhosis impairs 5-year survival (ANRS CO12 CirVir prospective cohort). *Gut,* 2017, vol. 66, 330-341
- **COSTENTIN CE ; LAYESE R ; BOURCIER V ; CAGNOT C ; MARCELLIN P ; GUYADER D et al.** Compliance With Hepatocellular Carcinoma Surveillance Guidelines Associated With Increased Lead-Time Adjusted Survival of Patients With Compensated Viral Cirrhosis: A Multi-Center Cohort Study. *Gastroenterology,* 2018, vol. 155, 431-442 e410
- **ALLAIRE M ; NAHON P ; LAYESE R ; BOURCIER V ; CAGNOT C ; MARCELLIN P et al.** Extrahepatic cancers are the leading cause of death in patients achieving hepatitis B virus control or hepatitis C virus eradication. *Hepatology,* 2018, vol. 68, 1245-1259
- **CACOUB P ; NAHON P ; LAYESE R ; BLAISE L ; DESBOIS AC ; BOURCIER V et al.** Prognostic value of viral eradication for major adverse cardiovascular events in hepatitis C cirrhotic patients. *American heart journal,* 2018, vol. 198, 4-17
- **GANNE-CARRIE N ; NAHON P ; CHAFFAUT C ; N'KONTCHOU G ; LAYESE R ; AUDUREAU E et al.** Impact of cirrhosis aetiology on incidence and prognosis of hepatocellular carcinoma diagnosed during surveillance. *JHEP Rep,* 2021, vol. 3, 100285
- **NAHON P ; BOURCIER V ; LAYESE R ; AUDUREAU E ; CAGNOT C ; MARCELLIN P et al.** Eradication of Hepatitis C Virus Infection in Patients With Cirrhosis Reduces Risk of Liver and Non-Liver Complications. *Gastroenterology,* 2017, vol. 152, 142-156 e142
- **PARK SH ; KIM S.** Pattern discovery of multivariate phenotypes by association rule mining and its scheme for genome-wide association studies. *Int J Data Min Bioinform,* 2012, vol. 6, 505-520
- **WOLBERS M ; BLANCHE P ; KOLLER MT ; WITTEMAN JC ; GERDS TA.** Concordance for prognostic models with competing risks. *Biostatistics,* 2014, vol. 15, 526-539
- **VICKERS AJ ; ELKIN EB.** Decision curve analysis: a novel method for evaluating prediction models. *Med Decis Making,* 2006, vol. 26, 565-574
- **VICKERS AJ ; VAN CALSTER B ; STEYERBERG EW.** Net benefit approaches to the evaluation of prediction models, molecular markers, and diagnostic tests. *BMJ,* 2016, vol. 352, i6
- **SHERMAN M.** HCC Risk Scores: Useful or Not?. *Seminars in liver disease,* 2017, vol. 37, 287-295
- **INNES H ; MORLING JR ; BUCH S ; HAMILLV ; STICKEL F ; GUHA IN.** Performance of routine risk scores for predicting cirrhosis-related morbidity in the community. *J Hepatol,* 07 March 2022, (22), 00129-5
- **DEGASPERI E ; GALMOZZI E ; PELUSI S ; D'AMBROSIO R ; SOFFREDINI R ; BORGHI M et al.** Hepatic Fat-Genetic Risk Score Predicts Hepatocellular Carcinoma in Patients With Cirrhotic HCV Treated With DAAs. *Hepatology,* 2020, vol. 72, 1912-1923
- **INNES H ; NISCHALKE HD ; GUHA IN ; WEISS KH ; IRVING W ; GOTTHARDT D et al.** The rs429358 Locus in Apolipoprotein E Is Associated With Hepatocellular Carcinoma in Patients With Cirrhosis. *Hepatol Commun,* 2022, vol. 6, 1213-1226
- **JAMIALAHMADI O ; MANCINA RM ; CIOCIOLA E ; TAVAGLIONE F ; LUUKKONEN PK ; BASELLI G et al.** Exome-Wide Association Study on Alanine Aminotransferase Identifies Sequence Variants in the GPAM and APOE Associated With Fatty Liver Disease. *Gastroenterology,* 2021, vol. 160, 1634-1646